# EUROPÄISCHE PATENTSCHRIFT

(11) **EP 2 696 910 B1**
(45) Veröffentlichungstag und Bekanntmachung des Hinweises auf die Patenterteilung: **01.06.2016**
(21) Anmeldenummer: 12715837.6
(22) Anmeldetag: 12.04.2012
(51) Int. Cl.: A61M 1/16, A61J 1/20, A61J 1/10

(54) **MEHRKAMMERCONTAINER ZUR HERSTELLUNG MEDIZINISCHER LÖSUNGEN**
MULTI-CHAMBER CONTAINER FOR PRODUCING MEDICAL SOLUTIONS
RÉCIPIENT À PLUSIEURS CHAMBRES POUR LA PRODUCTION DE SOLUTIONS MÉDICALES

(30) Priorität: 14.04.2011 DE 102011017048; 14.04.2011 US 201161475405 P
(43) Veröffentlichungstag der Anmeldung: 19.02.2014
(73) Patentinhaber: Fresenius Medical Care Deutschland GmbH, 61352 Bad Homburg (DE)
(72) Erfinder: KUGELMANN, Franz, 66606 St. Wendel/Bliesen (DE); HOERMANN, Joern, 66265 Heusweiler (DE)
(74) Vertreter: Stolmár & Partner Patentanwälte PartG mbB
(86) Internationale Anmeldenummer: PCT/EP2012/001578
(87) Internationale Veröffentlichungsnummer: WO 2012/139753

(56) Entgegenhaltungen:
- EP-A1- 1 621 177
- WO-A1-00/57833
- FR-A1- 2 766 797
- US-A1- 2009 166 363
- US-B2- 7 544 301

## Beschreibung

Gegenstand der Erfindung ist ein Mehrkammerbeutel zur Aufnahme von Konzentraten und Herstellung einer medizinischen Lösung aus den Konzentraten, sowie ein Verfahren zur Herstellung einer medizinischen Lösung aus mehreren Konzentraten.

Niereninsuffiziente Patienten leiden unter eingeschränkter Funktion der Nieren, so dass eine notwendige Ausscheidung von harnpflichtigen Substanzen aus dem Körper des Patienten verhindert wird. Die Abscheidung von toxischen Metaboliten aus dem Patienten muss durch Blutreinigung in einer Dialysebehandlung erfolgen. In der Dialyse erfolgt eine Blutreinigung über eine Stoffaustauschmembran, die auf der einen Seite mit dem Patientenblut und auf der anderen Seite mit einer Reinigungsflüssigkeit in Kontakt kommt. Bei der Reinigungsflüssigkeit handelt es sich um sogenannte Dialyselösungen, die die zur Ausscheidung bestimmten Substanzen aufnimmt und aus dem Patientenblut abtransportiert. Im Allgemeinen bestehen Dialyselösungen aus einer wässrigen Zusammensetzung von physiologisch wichtigen gelösten Komponenten, zum Beispiel Elektrolyten, Puffern oder osmotisch aktiven Reagenzien, zum Beispiel Glukose.

In der Peritoneal Dialyse (PD) wird die Dialyselösung in das Peritoneum des Patienten infundiert. Das Bauchfell des Patienten dient dabei als Stoffaustauschmembran, durch die eine Reinigung des Blutes erfolgt. Der Stofftransport wird durch diffusive Transportprozesse von harnpflichtigen Substanzen von der Blutseite durch die Bauchfellmembran ins Peritoneum, das mit Dialyselösung gefüllt ist, bestimmt. Der Dialyselösung ist ein osmotisches Agens zugesetzt, so dass im Peritoneum ein höherer osmotischer Druck vorherrscht als im Blut. Aufgrund des osmotischen Druckgefälles erfolgt ein Wasserübergang durch die Membran in den Raum des Peritoneums. Im Laufe der Therapie oder gegen Ende der Therapie wird das Peritoneum entleert und die Dialyselösung verworfen.

In der Hämodialyse (HD) wird das zu reinigende Blut des Patienten durch einen extrakorporalen Blutkreislauf geführt und mit einer Stoffaustauschmembran in Kontakt gebracht. Die gegenüberliegende Seite der Stoffaustauschmembran wird mit Dialyselösung in Kontakt gebracht, so dass membrangängige Substanzen aus dem Blut durch Membranübergang mit der Dialyselösung abtransportiert werden können. Der Prozess des Stoffübergangs kann bei den gängigen HD Therapien durch diffusive oder konvektive Transportvorgänge erfolgen. Insbesondere durch konvektive Transportvorgänge ist eine Entwässerung des Patienten möglich, so dass im Allgemeinen eine Lösung für die HD Dialyse einen geringeren osmotischen Druck zur Stoffreinigung des Blutes aufweisen muss als Lösungen, die für PD Dialyse geeignet sind.

In beiden Fällen der HD und PD Dialyse enthalten die Dialyselösungen typische gelöste Stoffe wie zum Beispiel:
- Elektrolyte Na, K, Mg, Ca, um einen annehmbaren Elektrolythaushalt des Patienten aufrecht zu erhalten
- Puffer (zum Beispiel Bikarbonat, Acetat, Laktat)
- Glukose (oder andere osmotische Agenzien), als osmotisches Mittel in der Peritonealdialyse oder zur Aufrechterhaltung des Blutzuckspiegels während der Hämodialyse
- Säuren oder Salze von Säuren (zum Beispiel HCl bzw. Cl⁻, Essigsäure Zitronensäure), die eventuell zur Neutralisation basischer Teil-Dialyselösungen beitragen oder als Gegenionen im elektrochemischen Gleichgewicht vorliegen

Die für Dialyselösung verwendeten Substanzen sind im Allgemeinen nicht in einer gebrauchsfertigen Mischung lagerfähig, da die Substanzen eine gegenseitige Degradation verursachen können. Die geforderte Lagerstabilität einer Komponente kann Lagerbedingungen voraussetzen, die für andere Komponenten zur Degradation führt. Zum Beispiel ist Glukose, abhängig von der Konzentration in Lösung, nur in einem bestimmten sauren pH-Wert Bereich über längere Zeit lagerfähig, ohne dabei im übermäßigen Maß unerwünschten Degradationsvorgängen zu unterliegen. Gleichzeitig ist die in Dialyselösung häufig als Puffer verwendete Verbindung Natriumbikarbonat nicht unter solchen sauren Bedingungen lagerfähig, da Bikarbonat abhängig vom pH Wert zur Zersetzung neigt und CO₂ freisetzen kann. Unter Zersetzungsbedingungen ändert sich die Konzentration von Bikarbonat, was aus therapeutischer Sicht inakzeptabel ist. Der steigende CO₂ Partialdruck stellt zudem Anforderung an die medizinischen Dialysegeräte, die zu technischen Problemen führt.

Es sind eine Vielzahl von alternativen Zusammensetzungen, Lagerbedingungen und Darreichungsformen von Dialyselösungen oder Konzentraten bekannt, die eine lange Lagerung ermöglichen. Bekannt ist die Aufteilung der Lösungskomponenten in eine Kombination von Teillösungen oder Konzentraten, so dass nur verträgliche Komponenten einer Teillösung oder eines Teilkonzentrats zusammen gelagert werden. Für Lösungen der Peritoneal Dialyse wird gängigerweise eine erste Teillösung umfassend Glukose, das die Funktion des Osmotikums übernimmt, bei einem sauren pH Wert mit weiteren Elektrolyten, zum Beispiel Natrium, Kalzium, Magnesium gelagert. Eine weitere basische oder gepufferte Teillösung ist notwendig, um mit der ersten sauren Teillösung eine physiologische oder wenigstens für die Therapie gebrauchsfertige Mischlösung aus dem ersten Teil und dem zweiten Teil zu liefern. Der zweite Teil besteht dabei häufig aus einer Lösung oder einem Konzentrat von Natriumbikarbonat und Natriumchlorid. Die Teillösungen oder Konzentrate werden dabei in mehreren Behältern oder in mehreren Kammern eines Behälters vorgelegt. Die Teillösungen oder Teilkonzentrate liegen getrennt vor, so dass keine gegenseitige Beeinflussung entsteht. Unmittelbar vor Gebrauch der Dialyselösung werden die getrennten Teillösungen oder Teilkonzentrate eventuell unter Zugabe weiterer wässriger Komponenten gemischt und für die die Behandlung bereitgestellt.

In der Hämodialyse werden die Teillösungen oder Teilkonzentrate oftmals in der Dialysemaschine vor und während des Verlaufs der Behandlung gemischt und zu einer fertigen Dialyselösung hergestellt. Hierfür werden oft Teilkonzentrate in fester oder in flüssiger Form verwendet, die in einzelnen Behältern vorliegen und durch einen Anschluss an die Dialysemaschine mithilfe eines vorbereiteten Hydrauliksystems verdünnt, gemischt und zur fertigen verwendbaren Dialyselösung aufbereitet werden.

Andere Entwicklungen in der Dialyse gehen dazu über, die notwendigen Konzentrate in einem einzigen Behälter vorzulegen. Zum einen vereinfacht dies die Herstellung und Handhabung der Behälter, zum anderen wird dadurch auch das hydraulische System der Dialysemaschine vereinfacht, da mitunter nur eine Aufnahmeeinheit für die Teillösungen oder Konzentrate vorzusehen ist und weniger Anschlüsse notwendig sind, um die Lösung durch das hydraulische System aufzubereiten. Dieser Trend kann vor allem in der Akutdialyse beobachtet werden, da hier eine größere Mobilität der Behandlungssysteme gefragt ist.

In einer weiteren Variante werden Dialyselösungen für die Hämodialyse nicht während des Verlaufs der Behandlung aus Konzentraten hergestellt, sondern das gesamte benötigte Volumen der Dialyselösung wird in einem Batch in einem der Behandlung vorgelagerten Schritt aufbereitet. Das Batch wird in einem Tank bevorratet, der dafür vorbereitet ist, mit einer Dialysemaschine verbunden zu werden. In manchen Fällen ist der Tank ein integraler Bestandteil einer Dialysebehandlungseinheit, oder kann in bestimmten Fällen auch wieder von diesem separat bewegt werden. Die Batchdialyse kann somit den Vorteil aufweisen, durch eine einmalige Zubereitung des Batches den Behandlungsplatz relativ ortsunabhängig zu wählen. Somit sind Behandlungsstationen an verschiedenen Orten einsetzbar, ohne auf eine Zubereitungseinheit von Dialyselösung oder einem Wasseranschluss, der das notwendige Wasser zur Verdünnung der Konzentrate liefert, angewiesen zu sein. In diesen Fällen wird die Dialyselösung an einer dafür vorgesehenen Einrichtung aus Konzentraten zusammengemischt und in einem meist mobilen Tank bevorratet.

Es ist bekannt, die notwendigen Konzentrate für die Herstellung eines Dialyselösungs-Batches in nur einer Gebindeeinheit vorzulegen. So sind beispielsweise Trockenkonzentrate bekannt, deren Granulatpartikel mehrschichtig oder mehrkomponentig aufgebaut sind. Jede Schicht eines Granulates oder jede Komponente enthält dabei die Substanzen, die für die Herstellung der Dialyselösung notwendig sind.

In einer weiteren Entwicklung werden verschiedene Trockenkonzentrate in einer Gebindeeinheit vorgelegt. Verschiedene Granulate, die unterschiedlichen Lösungskomponenten entsprechen, werden dabei schichtweise in einem Behälter gefüllt. Die Komponenten in einer Schicht liegen so im Wesentlichen getrennt von den Komponenten der nächsten Schicht vor. Sie sind dabei nur durch die Grenzflächen zwischen zwei benachbarten Schichten mit Komponenten der benachbarten Schichten in Kontakt.

Es hat sich bei der Überprüfung dieser Entwicklungen jedoch herausgestellt, dass selbst bei einem derartigen schichtartigen Aufbau eines Granulatpartikels oder bei einer geschichteten Vorlage von Trockenkonzentraten entsprechender Lösungskomponenten, nachteilige Interaktionen der unterschiedlichen Konzentrate auftreten können.

Trockenkonzentrate beanspruchen gegenüber Teillösungen oder lösungsartigen Teilkonzentraten in der Lagerung erhöhte Anforderungen. Die Trockenkonzentrate müssen während ihrer gewährleisteten Lagerungsdauer nicht nur den Aspekt der Degradation von Teilen der Konzentrate der Lösungskomponenten überwinden, sie müssen auch weiterhin den Aspekt einer guten Lösbarkeit mit einem Verdünnungsmittel erfüllen können. Beobachtungen des Standes der Technik in der Lagerung von Trockenkonzentraten haben gezeigt, dass partikelförmige Konzentrate unter Feuchtigkeitseinwirkung agglomerieren können. Das Konzentrat ist in einem agglomerierten Zustand nicht gut mit einem weiteren wässrigen Verdünnungsmittel mischbar, so dass die Lösungszeiten solcher Konzentrate im klinischen Gebrauch nicht akzeptiert werden können. Vorraussetzung ist also, dass Konzentrate, insbesondere wenn es sich um Trockenkonzentrate handelt, schnell mit Verdünnungsmittel oder anderen Lösungen durchmischbar sind.

Ein Vollkonzentrat, das alle für die Dialyse benötigten Bestandteile beinhaltet, ist in der Regel nicht lagerstabil. Das Konzentrat wird daher in Teilkonzentrate getrennt, so dass in jedem Teilkonzentrat nur miteinander lagerstabile und verträgliche Lösungskomponenten vorliegen. Ein für die Herstellung von medizinischen Lösungen vorbereitetes Set von Konzentraten oder Teillösungen kann somit aus zwei, drei oder mehreren Teilkonzentraten bzw. Teillösungen bestehen, die in einem Container oder Beutel gelagert werden.

EP 1 458 433 zeigt einen Konzentratbehälter, in dem die Lösungskomponenten oder Teilkonzentrate schichtweise angeordnet sind und somit miteinander unverträgliche Komponenten voneinander separiert werden.

EP 1 059 083 zeigt ein Granulat, deren Körner schichtweise aufgebaut sind. Damit werden einander unverträgliche Lösungskomponenten durch lösliche Pufferschichten voneinander getrennt.

WO 2007/144427 zeigt ein System zur Herstellung von Dialyselösungen mit einem Mehrkammerbeutel, der jeweils in einer Kammer ein Teilkonzentrat beinhaltet. Durch Zuführung eines Fluids werden die Trennlinien zwischen den Kammern aufgebrochen und die Dialyselösung hergestellt.

US 4,386,634 zeigt einen großvolumigen Behälter, in dem Trockenkonzentrate für die Herstellung einer Dialyselösung vorgelegt sind. Durch Zuleiten von Wasser wird ein flüssiges Konzentrat vorbereitet.

US 7,544,301 und EP 0 846 470 zeigen Verfahren zur Überwachung des Lösungsvorganges bei der Herstellung eines Batches einer Dialyselösung durch elektrische Leitfähigkeitsmessungen.

US 2009/0166363 offenbart einen Mehrkammerbeutel mit mehreren Konzentratkammern sowie einer Füllkammer.

Im Stand der Technik ist beschrieben, verschiedene Teilkonzentrate oder Teillösungen für die Herstellung von Dialyselösungen in einem Mehrkompartimentenbehälter vorzulegen. Mit der Lagerung der Lösungsbestandteile, aufgeteilt auf verschiedene Teilkonzentrate oder Teillösungen, kann eine ausreichende Lagerstabilität erreicht werden. Die Teilkonzentrate oder Teillösungen werden dabei in unterschiedlichen Behältern oder in unterschiedlichen Kammern eines Behälters gelagert. Die Kammern sind dabei durch Trennmittel voneinander getrennt. Durch Lösen der Trennmittel werden die Inhalte freigesetzt und ergeben zum Beispiel durch wässrige Verdünnung oder einfaches Durchmischen eine fertig anwendbare medizinische Lösung.

Für die Überwachung des Herstellprozesses von medizinischen Lösungen ist es wichtig Klarheit darüber zu haben, ob alle Teilkonzentrate im Prozess gemischt wurden. Im Allgemeinen ist der Lösungsprozess und/oder Verdünnungsprozess der Teilkonzentrate einfach durch Messung der elektrischen Leitfähigkeit zu überwachen. Enthalten die Teilkonzentrate ionische Substanzen, so liefern sie beim Lösen und/oder Verdünnen einen Beitrag zur elektrischen Leitfähigkeit. Im Einzelnen kann durch Erreichen eines endgültigen Leitfähigkeitswerts im Lösungs-/-Verdünnungsprozess ein Ende des Lösungsprozesses angezeigt sein. Es kann somit sicher darauf geschlossen werden, dass alle Teilkonzentrat- oder Lösungsteile gemischt wurden und keine weiteren Teilkonzentrate oder Teillösungen ungelöst und/oder ungemischt vorliegen. Analog zeigt ein Leitfähigkeitswert, der nicht einem zuvor definierten endgültigen Leitfähigkeitswert entspricht, an, dass eventuell ein Teilkonzentrat oder Teillösung nicht verdünnt und/oder gemischt wurde. Eine Überwachungseinheit kann somit in einen Aktionsmodus versetzt werden, so dass zum Beispiel ein Alarm oder weitere Lösungsprozessschritte eingeleitet werden.

Die Leitfähigkeitsmessung kann jedoch ungenügende Aussagen machen, wenn einzelne Konzentrate nur einen sehr geringen oder keinen Beitrag zur Leitfähigkeit aufweisen. Im ungünstigsten Fall kann bereits ein endgültiger Leitfähigkeitswert im Herstellprozess der Lösung angezeigt werden, obwohl Lösungsbestandteile, die keinen Beitrag zur elektrischen Leitfähigkeit aufweisen, ungelöst, ungemischt oder nicht verdünnt sind. Lösungskomponenten, die für medizinische Lösungen verwendet werden und nur einen geringen oder keinen Beitrag zur elektrischen Leitfähigkeit einer wässrigen Mischlösung beitragen können, zum Beispiel organische Substanzen sein:
- Wirkstoffe, Arzneistoffe
- insbesondere im Dialysebereich Osmotika: Glukose, Fruktose, Galaktose, Aminosäuren, Säuren: Essigsäure, Zitronensäure, Milchsäure, Bernsteinsäure, Fumarsäure, Oxalsäure, Äpfelsäure.

Unter einer geringen elektrischen Leitfähigkeit sind in diesem technischen Zusammenhang wässrige Lösungen zu verstehen, die einen Leitfähigkeitswert von 0,5 mS/cm und darunter aufweisen. Oder es sind solche Kompositionen, zum Beispiel Konzentrate mit oder ohne Verdünnungsmittel oder Teillösungen oder Suspensionen darunter zu verstehen, die in einer zu erwartenden Lösung nach Verdünnung eine Leitfähigkeitsänderung von 0,5 mS/cm und darunter verursachen. In diesem Sinne sind auch die oben genannten organischen Säuren als Substanzen zu bezeichnen, die je nach Konzentrierung diesen geringen Leitfähigkeitsbeitrag zur gesamten Lösung liefern.

Somit liegt das Problem vor, durch Leitfähigkeitsmessungen den Lösungsprozess von mehreren Konzentraten, Lösungen oder Teillösungen zu überwachen, wobei wenigstens eines der Konzentrate aus Substanzen besteht, die keinen Beitrag zur elektrischen Leitfähigkeit in der gebrauchsfertigen Lösung liefern.

### Beschreibung der Erfindung

Der Erfindung liegt die Aufgabe zugrunde, ein medizinisches Verfahren und einen medizinischen Container derart weiterzubilden, dass bei Herstellung einer medizinischen Lösung aus einem Verdünnungsmittel und mehreren Teilkonzentraten, von denen wenigstens ein Teilkonzentrat keinen oder nur einen geringen Beitrag zur Leitfähigkeit aufweist, durch Feststellen eines endgültigen Leitfähigkeitswertes plausibel darauf geschlossen werden kann, dass alle Teilkonzentrate oder Teillösungen zu einer gebrauchsfertigen Lösung gelöst oder vermischt wurden.

Die Aufgabe wird durch den Gegenstand von Anspruch 1, einen Container zur Lagerung und Herstellung einer medizinischen Lösung aus mehreren Konzentraten oder Teillösungen, sowie dem Verfahren nach Anspruch 16 , zur Herstellung einer gebrauchsfertigen medizinischen Lösung unter Verwendung des erfindungsgemäßen Containers gelöst. Bevorzugte Ausführungsformen werden durch die Merkmale der Unteransprüche dargestellt.

Demnach wird in einem erfindungsgemäßen Lösungsprozess in einen MehrkammerContainer ein Verdünnungsmittel in eine Füllkammer eingeleitet. Der Container enthält zudem mehrere Konzentratkammern, die Teilkonzentrate für die Herstellung der gebrauchsfertigen Lösung beinhalten. Im Laufe der Befüllung werden die Konzentratkammern aufgebrochen und Teilkonzentrate vermischen sich mit dem zuströmenden Verdünnungsmittel. Im Laufe des Lösungsprozesses kommt es zu einer Änderung der elektrischen Leitfähigkeit, die durch eine entsprechende Messvorrichtung aufgenommen werden kann. Durch Anordnung der Konzentratkammern und Zuflussports für das einströmende Verdünnungsmittel und der gewählten Geometrie des Containers, kann es zu einer vorbestimmten Abfolge der Freisetzung der Teilkonzentrate führen. Dabei wird eine Konzentratkammer mit einem Teilkonzentrat, das keinen oder einen nur geringen Beitrag zur elektrischen Leitfähigkeit der gebrauchsfertigen Lösung beiträgt, zeitlich vor oder zeitgleich mit einer Konzentratkammer aufgebrochen, die ein Teilkonzentrat enthält, dass einen Beitrag zur elektrischen Leitfähigkeit der gebrauchsfertigen Lösung liefert. Durch Überwachung der elektrischen Leitfähigkeit der Lösungsherstellung, die mit der Freisetzung der Teilkonzentrate, die einen Beitrag zur elektrischen Leitfähigkeit der gebrauchsfertigen Lösung liefert, korreliert, wird durch die Freisetzung der Teilkonzentrate überwacht, die keinen oder einen nur geringen Beitrag zur elektrischen Leitfähigkeit der gebrauchsfertigen Lösung haben.

Weitere Einzelheiten und Vorteile der Erfindung werden durch die in den Zeichnungen dargestellten Ausführungsbeispiele näher beschrieben. Es zeigt:
Figur 1 einen erfindungsgemäßen Container mit drei Kammern, die Lösungskomponenten beinhalten.
Figur 2 einen erfindungsgemäßen Beutel Container mit sechs Kammern, die Lösungskomponenten enthalten.
Figur 3 einen erfindungsgemäßen Beutel, im gefüllten Zustand, nachdem alle Kammertrennlinien aufgetrennt wurden.
Figur 4 eine weitere Ausführung eines Containers mit drei Kammern, die Lösungskomponenten enthalten.
Figur 5 eine weitere Ausführung eines erfindungsgemäßen Containers, die Lösungskomponenten enthalten.
Figur 6 einen weiteren Container mit drei Kammern, die Lösungskomponenten enthalten.
Figur 7 einen erfindungsgemäßen Container mit vier Kammern.
Figur 8a schematisch in einem seitliche Querschnitt ein erfindungsgemäßes Containersystem mit einem inneren Beutel, der Konzentratkammern aufweist und einem äußeren Beutel, wobei die erste Folie des inneren Beutels tief gezogene Bereiche aufweist.
Figur 8b schematisch in einem seitlichen Querschnitt ein erfindungsgemäßes Containersystem mit einem inneren Beutel und einem äußeren Beutel, wobei die erste Folie des inneren Beutels tief gezogenen Bereiche aufweist und die zweite Folie nicht tief gezogen ist und wobei die Konzentratkammern des inneren Beutels bereits geöffnet sind.
Figur 8c ein erfindungsgemäßes Containersystem aus einem inneren Beutel und einen umhüllenden Beutel, wobei die Folienwandungen des inneren Beutels gegenüberliegende tief gezogene Bereiche aufweisen und die Konzentratkammern des inneren Beutels bereits geöffnet sind.

In der vorliegenden Erfindung werden ein Mehrkammercontainer (1, 201, 401,501, 601, 701) und ein Verfahren zur Herstellung einer medizinischen Lösung, insbesondere einer Dialyselösung beschrieben. Ausgehend von Teilkonzentraten (A, B, C, C1, C2) oder Teillösungen wird eine gebrauchsfertige medizinische Lösung durch Mischen von Teillösungen und/oder durch Zudosieren eines wässrigen Verdünnungsmittels hergestellt.

Der erfindungsgemäße Container enthält wenigstens eine Füllkammer (10, 210, 410, 510, 610, 710) und mindestens zwei weitere Kammern (3, 4, 5, 203 a, 203b, 204a, 204b, 205a, 205b, 403, 404, 405, 503, 504, 505, 603, 604, 605, 703a, 703b, 704, 705), die Konzentrate (A, B₁, B₂, C, C₁, C₂), Teilkonzentrate oder Teillösungen enthalten, die bei Lagerung miteinander inkompatibel sind. Die Kammern werden im Folgenden als Konzentratkammern bezeichnet, wobei darunter zu verstehen ist, dass sie Konzentrate enthalten, die durch Mischen mit einem Verdünnungsmittel Teile für die Herstellung der gebrauchsfertigen medizinischen Lösung liefern. Unter einem Konzentrat werden in diesem Zusammenhang trockene Substanzen in Pulver-, Tabletten- oder Granulatform verstanden. Ebenso sind von dem Begriff Konzentrat auch Substanzen in Mischung mit Wasser umfasst, wobei diese in Suspension oder Emulsion, als konzentrierte flüssige Form oder in wässriger Lösung vorliegen können.

Im Folgenden ist unter dem Begriff Container jede Form eines Hohlgefäßes aus biegesteifen oder flexiblen Wandungsmaterialien zu verstehen, der insbesondere auch Beutel umfasst.

Im Allgemeinen sind unter einem Beutel Behälter zu verstehen, deren Wandungsmaterial aus flexiblem Material besteht, die unter dem Eigengewicht zusammenfallen und damit als kollabierbare Behälter gelten. Das Kollabieren eines Beutels ist als wesentliches strukturelles Merkmal des Beutels anzusehen. Insbesondere können Beutel durch die kollabierbare Charakteristik der Wandungen ohne Druckausgleich mit Fluiden befüllt oder entleert werden.

Behälter mit halbsteifem Wandungsmaterial, die unter ihrem Eigengewicht zwar nicht zusammenfallen, aber durch Handkraft deformierbar sind, sind ebenso von dem Begriff "Container" umfasst.

Der erfindungsgemäße Container (1, 201, 401,501, 601, 701) oder Beutel kann aus zwei gegenüberliegenden Außenwandungen (2a, 2b, 202a, 202b, 402a, 402b, 502a, 502b, 602a, 602b, 702a, 702b) bestehen und zum Beispiel aus Folienmaterialien durch Schweißwerkzeuge oder andere Verfahren, zum Beispiel Extrusionsblasverfahren hergestellt werden. Schweißnähte können wenigstens abschnittsweise die äußere Kontur oder Umfassungslinie (8, 208, 408, 508, 608, 708) des Containers oder Beutel umschreiben und so einen inneren Raum mit einem vorbestimmten Fassungsvolumen definieren.

Bei dem Container (1, 201, 401,501, 601, 701) handelt es sich in einer bevorzugten Form um einen Mehrkammerbeutel aus flexiblen Folienabschnitten (2a, 2b, 202a, 202b, 402a, 402b, 502a, 502b, 602a, 602b, 702a, 702b). Dabei kann unter einem Beutel auch ein Container verstanden werden, der so ausgestaltet ist, dass Abschnitte des Containers aus einem biegsteifen Material bestehen, während weitere Abschnitte aus einem flexiblen Folienmaterial bestehen.

Der erfindungsgemäße Container beziehungsweise Beutel enthält Trennlinien (9, 9a, 9b, 209a, 209b, 209d, 209e, 209f, 409, 409a, 509, 509b, 609a, 609b, 609, 709a, 709b, 709c, 709d), die den Container in mehrere Kammern unterteilt. Die Trennlinien bestehen bevorzugt aus Schweiß- oder Adhäsivlinien, die gegenüberliegende Seiten eines Containers beziehungsweise Beutels miteinander verbinden. Semipermanente Abschnitte der Trennlinien sind so konstruiert, dass sie durch Kraft- oder Druckaufwendung lösbar sind, ohne die Umfassungswände des Containers zu beschädigen. Ebenso sind bei der Verwendung von biegesteifen Behältern von der Definition der semipermanenten Trennlinien auch Materialabschnitte umfasst, die durch Kraft- oder Druckaufwendung als Sollbruchstellen dienen und eine Fluid führende Passage zwischen zwei Kompartimenten freigeben. Alternativ können auch andere Trennlinien mit semipermanenten Abschnitten verwendet werden. Darunter sind ebenso Schweißlinien mit eingeschweißten sog. Brechkonnektoren zu verstehen, die durch Handkraft gelöst werden. Ebenso können eingeschweißte Folienstücke als Trennwände diese Trennlinie bilden, die wiederum so konstruiert sind, dass sie bei Kraftaufwendung zerbersten. Alternativ ist unter einer Trennlinie auch ein Mechanismus zu verstehen, der Folienabschnitte entlang einer Linie durch einen Klemmmechanismus oder Faltmechanismus zusammenhält und das ungewollte Vermischen der Kammerinhalte verhindert.

Die Trennlinien (9, 9a, 9b, 209a, 209b, 209d, 209e, 209f, 409, 409a, 509, 509b, 609a, 609b, 609, 709a, 709b, 709c, 709d) unterteilen den Mehrkammercontainer in wenigstens eine Füllkammer und wenigstens zwei Konzentratkammern. Dabei werden wenigstens eine erste Kammer und eine zweite Kammer durch wenigstens eine erste Trennlinie (9, 209a, 209d, 409, 509, 609, 709a, 709c) von der Füllkammer (10, 210, 410, 510, 610, 710) getrennt. Die eine erste Trennlinie kann abschnittsweise aus einer permanenten oder semipermanenten Trennlinie bestehen oder vollständig aus einer semipermanenten Trennlinie bestehen. Insbesondere kann sie mit einem Abschnitt einer permanenten Umfassungslinie des Containers zusammenwirken (8, 208, 408, 508, 608, 708). Die Konzentratkammern (3, 4, 5, 203 a, 203b, 204a, 204b, 205a, 205b, 403, 404, 405, 503, 504, 505, 603, 604, 605, 703a, 703b, 704, 705) werden dabei durch die semipermanente Trennlinie und Abschnitte der permanenten Umfassungslinie (8, 208, 408, 508, 608, 708) umschlossen und gegen die Füllkammer gegrenzt. Die Konzentratkammern sind so vollständig von einer Begrenzungslinie umschlossen, die in Abschnitten permanent, aus der Umfassungslinie (408, 608), oder semipermanent, aus der einen ersten Trennlinie (409, 609) bestehen kann.

Erfindungsgemäß ist die die Konzentratkammer umschließende Linie der einen ersten Kammer (4, 204a, 204b, 504, 704, 705) und der einen zweiten Kammer (3, 203a, 203b, 503, 703a, 703b), vollständig von der einen ersten wenigstens abschnittsweise semipermanenten Trennlinie (9, 209a, 209d, 509, 709a, 709c) umschlossen, ohne mit der Umfassungslinie (8, 208, 508, 708) des Containers zusammenzuwirken. Die eine erste Trennlinie der Kammern bildet dabei eine geschlossene Linie, die die eine erste Kammer und die eine zweite Kammer seitlich begrenzt. Zusätzlich sind Konzentratkammern durch die Außenwandungen (2a, 2b, 202a, 202b, 402a, 402b, 502a, 502b, 602a, 602b, 702a, 702b) des Behälters begrenzt, so dass die Kammern allseitig begrenzt sind.

Die eine erste Trennlinie (9, 209a, 209d, 409, 509, 609, 709a, 709c) kann dabei so beschaffen sein, dass sie in einem Abschnitt, in dem sie die eine erste Kammer (4, 204a, 204b, 404, 504, 604, 704, 705) und die eine zweite Kammer (3, 203a, 203b, 405, 503, 603, 703a, 703b) begrenzt, vollständig semipermanent ausgestaltet ist. Es tritt hier der Vorteil zutage, dass ein Lösen oder ein Bruch der Trennlinie in diesem Abschnitt durch Kraftaufwendung die Begrenzung beider Kammern aufhebt und die Kammerinhalte freigibt. Insbesondere ist es so, dass ein anfänglicher Bruch der semipermanenten Trennlinienabschnitte eine höhere Kraft erfordert, als ein weiteres Aufreißen der bereits an einer Stelle gebrochenen Trennlinie. Erfolgt ein Bruch der einen ersten Trennlinie in einem Abschnitt, der die eine erste Kammer begrenzt, wird mit großer Wahrscheinlichkeit auch der begrenzende Abschnitt der einen zweiten Kammer aufgebrochen.

Für den Herstellungsprozess der medizinischen Lösung in dem erfindungsgemäßen Container ist ein Zuführport (6, 206, 406, 506, 606, 706) vorgesehen, der in die Umfassungslinie (8, 208, 408, 508, 608, 708) des Containers eingearbeitet ist und die Füllkammer des Containers in Fluidverbindung mit einer äußeren Fluidquelle bringen kann.

Bei aufrechter Lagerung des Beutels wird zunächst durch einen Zuführport eine Verdünnungsflüssigkeit, zum Beispiel Wasser oder eine Teillösung in Füllkammer (10, 210, 410, 510, 610, 710) geleitet. Dadurch steigt der hydrostatische Druck, der auf die Begrenzungsebenen (2a, 2b, 202a, 202b, 402a, 402b, 502a, 502b, 602a, 602b, 702a, 702b) im Inneren des Containers wirkt, infolge der Befüllung und bewirkt zumindest ein Öffnen der ersten Trennlinie (9, 209a, 209d, 409, 509, 609, 709a, 709c). Der Inhalt der einen ersten Konzentratkammer (4, 204a, 204b, 404, 504, 604, 704, 705) wird mit dem zuströmenden Verdünnungsmittel oder alternativ mit einer zuströmenden Teillösung vermischt. Spätestens durch weiteres Füllen des Containers wird die erste Trennlinie weiter so geöffnet, dass auch der Inhalt der einen zweiten Kammer (3, 203a, 203b, 405, 503, 603, 703a, 703b) mit dem zuströmenden Verdünnungsmittel/Teillösung in Kontakt kommt und vermischt wird. Das über den Druckaufbau verursachte Öffnen der ersten abschnittsweise semipermanenten Trennlinie verursacht somit das Öffnen der einen ersten Kammer und der einen zweiten Kammer, entweder gleichzeitig oder sequentiell.

Die Füllkammer kann dabei in einer Ausführungsform die umschlossenen Konzentratkammern im Inneren des Beutels umgeben, so dass beim Aufbrechen der ersten oder zweiten Konzentratkammer die Konzentrate direkt in die mit Verdünnungsmittel befüllte Verdünnungskammer gelangen. Bevorzugt ist die Füllkammer im Ausgangszustand, eine Leerkammer, die in direkter Fluidverbindung mit dem Zuführport steht.

Bevorzugt ist der semipermanente Abschnitt der Trennlinie so ausgestaltet, dass sich die Trennlinie allein durch den hydrostatischen Druck der Befüllung öffnet ohne dass weitere Kraftaufwendung notwendig ist.

Es ist somit weitgehend sichergestellt, dass beide Kammerinhalte mit dem zuströmenden Verdünnungsmittel/Teillösung in Kontakt kommen und zu einer medizinischen Lösung vermischt werden. Somit kann auch weitgehend sicher sein, dass der Kammerinhalt einer ersten Kammer, die Konzentrate beinhaltet, die nicht zur elektrischen Leitfähigkeit beitragen, vollständig in die Lösung eingebracht ist, wenn der Lösungsprozess eines Kammerinhaltes einer zweiten Kammer, die Komponenten beinhaltet, die zur Leitfähigkeit beitragen, durch Leitfähigkeitsmessungen bestätigt wird.

Der Container kann weiterhin eine weitere dritte Konzentratkammer (5, 205a, 205b, 403, 505, 605) umfassen. Dies ist insbesondere dann von Vorteil, wenn die Lösungskomponenten aus Stabilitätsgründen wenigstens in drei Konzentratteile getrennt werden müssen. Eine dritte Konzentratkammer kann dabei im Container so angeordnet sein, dass die eine erste Konzentratkammer (4, 204a, 204b, 504, 604), die eine zweite Konzentratkammer (3, 203a, 203b, 503, 603) und die eine dritte Konzentratkammer (5, 205a, 205b, 505, 605) von der einen ersten Trennlinie (9, 209a, 209d, 509, 609) umschlossen sind oder die Kammern in Zusammenwirkung mit der äußeren Umfassungslinie (8, 208, 508, 608) umschlossen sind. Ein sich aufbauender hydrostatischer Druck beim Befüllen der Füllkammer verursacht ein Aufbrechen der wenigstens abschnittsweise semipermanenten ersten Trennlinie und ein Freisetzen der Kammerinhalte (A, B, C, C₁, C₂) der einen ersten Konzentratkammer, der einen zweiten Konzentratkammer und der einer dritten Konzentratkammer. Das Auflösen eines Konzentrates einer der drei Kammern, bestehend aus Lösungskomponenten, die keinen Beitrag zur elektrischen Leitfähigkeit beitragen, wird somit durch Leitfähigkeitsmessung über den Verdünnungs-/Mischprozess der anderen Kammern, die einen Beitrag zur elektrischen Leitfähigkeit haben, mit überwacht. Die Trennlinie ist dabei zumindest in einem Bereich, der gleichzeitig alle drei Kammern von der Füllkammer abtrennt, semipermanent.

Vorzugsweise handelt es sich bei dem Container um einen Beutel, der aus Folienmaterial hergestellt ist. Dabei können Abschnitte aus einem biegesteifen Material hergestellt sein. Eine alternative Ausführung sieht vor, dass eine Seitenwand des Beutels aus einem biegesteifen Material hergestellt ist. Die biegesteife Seitenwand verleiht dem Beutel eine stützende Struktur. Die biegesteife Seitenwand kann insbesondere bei großvolumigen Beuteln von Vorteil sein und dem Beutel mehr Stabilität verleihen. Vorzugsweise wird der Beutel aus Kunststoffmaterialien hergestellt, insbesondere aus thermoplastischen und thermoplastisch elastomeren Kunststoffmaterialien. Bevorzugte Polymertypen sind Polyolefine und Polyalphaolefine, sowie hydrierte Styrolblockcopolymere. In diesem Zusammenhang werden bevorzugt Polymertypen wie Polypropylen, Poylethylen, Copolymere von Ethylen, Propylen, Buthen, Hexen oder Octen, Styrolisoprenstyrol Blockcopolymere (SIS), Styrolethylenbuthylenblockcopolymere (SEBS), Styrolethylenpropylenblockcopolymere (SEPS) und weitere verwendet.

Besteht der Container aus einem Beutel, bieten sich Vorteile für die Konstruktion der Trennlinien. Es ist bekannt, dass Mehrkammerbeutel durch permanente Umrandungsschweißnähte und peelbare Nähte im Kammertrennbereich hergestellt werden. Unter Peelnähten sind in diesem Zusammenhang Verbindungsstellen zweier Fügepartner zu verstehen, die durch Wärmebehandlung eine Adhäsivwirkung aufeinander ausüben. Im bevorzugten Fall handelt es sich bei den Fügepartnern um zwei gegenüberliegende Folienstücke eines Beutels, die im Schweißprozess mit einem Schweißbalken durch Wärmeeinwirkung und Anpressdruck miteinander verbunden werden. Die Schweißtemperatur bedingt, mit welcher Kraft die Peelnaht geöffnet werden kann. Unter einer Peelnaht ist eine Adhäsiwerbindung zu verstehen, die durch Kraftwirkung wieder gelöst werden kann, ohne dass ein vollständiger Bruch des Folienmaterials erfolgt. In besonderen Ausführungen kann unter Peelverbindung auch eine solche Verbindung verstanden werden, die durch Krafteinwirkung eine teilweise Delamination eines mehrschichtigen Folienverbundes verursacht. In diesen Fällen ist es wichtig, das der Delaminationsriss keinen vollständigen Bruch des Folienmaterials verursacht, was den Beutel unbrauchbar machen würde.

Im vorliegenden Fall sind alternative Ausführungen für die eine erste Trennlinie im erfindungsgemäßen Mehrkammerbeutel vorgesehen. In einer ersten Ausführung besteht die Trennlinie abschnittsweise aus peelbaren und damit semipermanenten Trennlinien. Das Öffnen der Peelnähte unter Kraftaufwendung oder durch Flüssigkeitsdruck bewirkt ein Freisetzen und ein folgendes Vermischen der Kammerinhalte mit eingeströmten Verdünnungsmitteln oder Teillösungen. Permanente Abschnitte im Trennlinienbereich verbinden die Wandungen des Beutels selbst im gefüllten Zustand. Dies kann dem Beutel Stabilität verleihen, da ein Berstdruck, der bei befüllten Beutel auf die Umrandungsschweißnähte wirkt, verringert wird. Insbesondere bei großvolumigen Beuteln größer 5L ist dies für die Stabilität des Beutels von Bedeutung.

In einer weiteren Ausführung sind die Trennlinien vollständig aus peelbaren Nähten hergestellt. Ein Lösen der Nähte beim Befüllen des Beutels ermöglicht ein gutes Durchmischen aller Kammerinhalte und eine vereinfachte produktionstechnische Herstellung der Trennlinien.

Der Peelcharakter der Trennlinien wird dabei durch den Schweißprozess beeinflusst. Je nach Temperatur des Schweißwerkzeuges können Peelnähte hergestellt werden, die mit einer geringeren Kraft lösbar sind. Bei einer höheren Temperatur des Schweißwerkzeuges entstehen Peelnähte mit höherer Festigkeit. Peelnahtfestigkeiten werden nach bekannten Methoden und Normen bestimmt. Die Peelnahtfestigkeit wird geläufig durch einen T-peel-Test bestimmt. Hierbei werden zwei Folien-Teststreifen übereinander gelegt und an einem Abschnitt verschweißt. Die losen Streifenenden werden jeweils in Greifbacken einer Prüfmaschine eingespannt, so dass der Teststreifenverbund ein T beschreibt. Anschließend wird an dem Folienverbund ein Zugversuch ausgeführt. Der Zugversuch kann in Anlehnung an die EN ISO 527-3 erfolgen. Ebenso zeigen die Normen ASTM F 88 - 07, ASTM D 1876 - 01 Methoden zur Bestimmung der Peelnahtfestigkeit.

Für den Peelcharakter einer Trennlinie, die bei einer definierten Temperatur des Schweißwerkzeuges hergestellt wurde, ist darüber hinaus die Komposition der Folienschichten von Bedeutung, die durch die Wärmebehandlung miteinander verbunden werden. Insbesondere hat sich eine Mischung aus einem Thermoplastischen Polymer und einem elastomeren Polymer bewährt. Beispielhafte Ausführung ist eine Zusammensetzung von 80 % eines thermoplastischen Polypropylens (PP) und 20% eines hydrierten Styrolblockcopolymeren, wie zum Beispiel einem thermoplastischen Elastomeren aufgebaut aus Polymerblöcken von Styrol und Ethylen-Butylen (SEBS). Die heterophasige Mischung der Polymere liefert bei niedrigen Temperaturen peelbare Schweißlinien und bei höheren Temperaturen permanente Schweißlinien, die nur unter Folienbruch lösbar sind.

Je nach Anwendungsfall eines erfindungsgemäßen Beutels können unterschiedliche Peelnahtstärken erforderlich sein. In einem Fall kann ein erfindungsgemäßer Mehrkammerbeutel mit flüssigem Konzentrat in einer der Kammern vorliegen und der Beutel dafür vorgesehen sein, dass die Peelnähte durch Anwendung eines äußeren Drucks geöffnet werden. In einem solchen Fall wird durch Handdruck oder Rollen des Beutels Druck auf die Flüssigkeit in einer Kammer ausgeübt, so dass der entstehende Flüssigkeitsdruck die angrenzende Peelnaht aufbricht. Ein solcher Beutel benötigt in der Regel eine höhere Peelnahtfestigkeit, da durch Transporterschütterungen bereits Druck auf die Flüssigkeit in einer Kammer ausgeübt werden kann, so dass die angrenzende Peelnaht beschädigt werden kann. Peelnahtstärken können in diesem Fall sein:
- 2-20 N/15mm, bevorzugt 2- 15 N/15mm, bevorzugt 2-10 N/15mm, 3- 8 N/15mm, 2-5 N/15 mm

Werden Mehrkammerbeutel mit festen Konzentraten in einer oder mehreren der einzelnen Kammern verwendet, besteht die Gefahr des ungewollten Öffnens der Peelnähte nicht, da sich ein äußerer Druck durch zum Beispiel granulatförmige oder pulverförmige Konzentrate innerhalb der Kammer nicht fortsetzt und auf die Trennlinien einwirkt. Es können generell niedrigere Peelnahtfestigkeiten gewählt werden. Insbesondere gibt es Anwendungen von Mehrkammerbeuteln, bei denen die Peelnähte durch einen inneren Druck einer zuströmenden Flüssigkeit gelöst werden. In solchen Fällen ist ebenfalls eine niedrige Peelnahtstärke vorteilhaft, so dass sich die Konzentrat-Kammern unproblematisch aufbrechen lassen und eine vollständige Mischung der Inhalte mit der zuströmenden Flüssigkeit erfolgt. In solchen Anwendungen sind Peelnahtstärken von
- 0,1- 6 N/15mm, bevorzugt 0,2- 5 N/15 mm, bevorzugt 0,2- 4 N/15 mm, bevorzugt 0,3-2 N/15 mm
vorteilhaft.

Aus Gründen der Stabilität können unterschiedliche Teilkonzentrate zur Herstellung medizinischer Lösungen zum Beispiel in drei Konzentratkammern gelagert werden. Ebenso können die Lösungsbestandteile medizinischer Lösungen jeweils einzeln in einer eigenen Konzentratkammer gelagert werden. So kann eine Vielzahl von Kammern vorliegen, entsprechend der Anzahl an notwendigen Lösungskomponenten. Weiterhin kann es aus Gründen der Lösungsherstellung, zum Beispiel schnelleres Auflösen mehrerer kleiner Konzentrate, vorteilhaft sein, die Konzentrate in mehr als zwei Kammern zu lagern.

Erfindungsgemäß sind wenigstens zwei der Konzentratkammern von einer ersten Trennlinie gegen die Füllkammer begrenzt. Alternativ können auch wenigstens drei Kammern von einer ersten Trennlinie gegen die Füllkammer begrenzt sein. Alternativ können auch vier Kammern von einer ersten Trennlinie gegen die Füllkammer begrenzt sein. Optional können eine fünf, sechs, sieben, acht Konzentratkammern durch die eine erste Trennlinie gegen die Füllkammer begrenzt sein. Bevorzugt ist eine Ausführung, bei der wenigstens zwei oder drei oder vier der vorab genannten Konzentratkammern von der einen ersten Trennlinie entlang einer geschlossenen Linie umschlossen sind und gegen die Füllkammer begrenzt sind.

Dabei ist es vorteilhaft, wenn wenigstens zwei Konzentratkammern (4, 204a, 204b, 404, 504, 604, 704, 705, 3, 203a, 203b, 405, 503, 603, 703a, 703b), die von der einen ersten Trennlinie (9, 209a, 209d, 509, 709a, 709c) gegenüber der Füllkammer begrenzt sind, untereinander durch eine zweite Trennlinie (9b, 209g, 209f, 409a, 509b, 609b, 709b, 709d) voneinander getrennt werden. Dabei kann die zweite Trennlinie als permanente Schweißnaht oder als semipermanente oder in Beutelausführungsformen als peelbare Schweißnaht vorliegen. Im ersten Fall verbleibt nach Lösen der ersten Trennlinie ein Schweißnahtsteg, der die kammertrennende zweite Trennlinie bildet, die zur Stabilisierung des gefüllten Beutels beitragen kann. Im zweiten Fall wird auch die peelbare zweite Trennlinie durch Druckanwendung, zum Beispiel äußerer Druckanwendung oder innerer Fülldruck, getrennt, so dass die gegenüberliegenden Folienblätter des Beutels nicht mehr miteinander verbunden sind.

Alternativ kann auch eine erste, eine zweite und eine dritte Konzentratkammer (4, 204a, 204b, 504, 604, 3, 203a, 203b, 503, 603, 5, 205a, 205b, 505, 605) von einer ersten Trennlinie gegenüber der Füllkammer begrenzt sein und zwischen der ersten Kammer und der zweiten Kammer eine zweite Trennlinie (9b, 209g, 209f, 409a, 509b, 609b) und zwischen der zweiten und der dritten Kammer eine dritte Trennlinie (9a, 209b, 209e, 509a, 609a) vorhanden sein. Die zweite und dritte Trennlinie können jeweils permanent oder semipermanent sein und in Beutelausführungen als permanente oder als peelbare Schweißnähte vorliegen. Im bevorzugten Fall sind dabei die eine erste, zweite und dritte Konzentratkammer von der einen ersten Trennlinie umschlossen. Und im besonders bevorzugten Fall sind alle Trennlinien semipermanente Trennlinien insbesondere Peelnähte.

Alternativ können, insbesondere in Ausführungsformen von erfindungsgemäßen Beuteln mit vier oder mehr Konzentratkammern, weitere Trennlinien so wie die erste Trennlinie konstruiert sein, um weitere Konzentratkammern gegenüber der Füllkammer zu begrenzen.

Die Konzentratkammern können dabei Konzentrate, Teilkonzentrate, flüssige Konzentrate und/oder Teillösungen enthalten. Zwei oder mehr Konzentratkammern von wenigstens vier Kammern können unterschiedliche oder gleiche Konzentrate, Teilkonzentrate, flüssige Konzentrate und/oder Teillösungen enthalten.

In einer bevorzugten Ausführungsform können die Trennlinien, also erste Trennlinie, zweite Trennlinie, dritte usw. Trennlinien in Beutelausführungen als Peelnähte und integral ausgebildet sein. D.h., dass die einzelnen semipermanenten Trennlinien nicht von einander abgesetzt sind, sondern ineinander übergehen oder überlappen. Peelnähte zeichnen sich dadurch aus, dass der Kraftaufwand für einen ersten Anriss der Peelnaht höher ist, als der Kraftaufwand für ein weiteres Trennen einer bereits angerissenen Peelnaht. Die integrale Konstruktion der Abschnitte der ersten Trennlinie, der zweiten Trennlinie, der dritten Trennlinie oder weiterer Trennlinien als zusammenhängende Peelnahtkonstruktion sichert ein Öffnen aller dieser peelbaren Abschnitte, da die Weiterreißfestigkeit einer angepeelten Peelnaht herabgesetzt ist. Findet ein Anpeelen an einem Abschnitt der Peelnahtkonstruktion statt, kann sichergestellt werden, dass die weiteren Abschnitte mit einem geringeren Kraftaufwand geöffnet werden.

Weiterhin weist der erfindungsgemäße Container einen Zuführport (6, 206, 406, 506, 606, 706) und/oder einen Entnahmeport auf. Der Zuführport ermöglicht das Einfüllen eines Fluids, wie zum Beispiel eines wässrigen Verdünnungsmittels zur Vermischung der Konzentrate, der Teilkonzentrate, der flüssige Konzentrate und/oder der Teillösungen zu einer medizinisch anwendbaren Lösung, zum Beispiel über eine externe Pumpe. Handelt es sich bei dem Container um einen Beutel, so entfaltet sich der Beutel durch das Einfüllen des Vedünnungsmittels. Der sich aufbauende innere hydrostatische Druck übt über die Folienwandung eine Zugbelastung auf die Peelnähte aus. Durch weiteres Befüllen lösen sich die erste Trennlinie, zweite Trennlinie, dritte Trennlinie usw. sukzessive nach dem Befüllungsgrad und hydrostatischem Druck im Inneren des Beutels und die Kammerinhalte werden freigegeben. Es erfolgt im weiteren Verlauf eine Vermischung der Kammerinhalte mit dem über den Port zuströmenden Verdünnungsmittel, so dass sich nach dem Lösungsprozess eine anwendbare medizinische Lösung bevorzugt eine Dialyselösung ergibt. Der Container weist in einer weiteren Ausführung einen Entnahmeport auf, mit dessen Hilfe die hergestellte Lösung für weitere Anwendungen entnommen werden kann. Bevorzugt gibt es nur einen Port, durch den Befüllung und Entnahme vorgenommen werden kann. Eine weitere Ausführungsform sieht vor, den Entnahme-/Befüllungsport als Schlauchstück zu gestalten, der die Außenwandungen des Containers durchdringt, zum Beispiel durch eine Einschweißstelle, und ins Innere des Beutels führt. Dabei ist es für maschinelle Befüllungsverfahren bevorzugt, dass der Container aufrecht gelagert wird, zum Beispiel an einer integralen Halteschiene (11, 211, 411, 511, 611, 711) hängend gelagert wird und der Schlauch des Zuführports im Inneren des Containers in den unteren Teil des Containers führt, so dass der Container von unten befüllt wird. Alternativ kann der Zuführport bei aufrechter Lagerung des Containers auch von unten an den Container angeschlossen sein und das Schlauchstück von untern in das Innere des Beutels hineinragen, um eine Befüllung von unten zu bewirken. Eine Befüllung von unten ist bei aufrechter Lagerung des Containers vorteilhaft, da beim Zuströmen eventuell durch weitere Mittel (6c, 206c, 406c, 506c, 606c, 706c) Turbulenzen erzeugt werden können, die eine Vermischung der Konzentrate, Teilkonzentrate, flüssige Konzentrate und/oder Teillösungen begünstigen.

Vorteilhaft weist der Container eine Füllkammer - bevorzugt eine leere Füllkammer - auf, in die der Zuführport hineinragt. Die Füllkammer wird zunächst gefüllt und mit ansteigendem Fülldruck werden die Trennlinien gelöst, so dass die Inhalte der Konzentratkammern mit einer bereits vorliegenden Flüssigkeitsmenge vermischt werden. Dies hat zum Vorteil, dass die Inhalte der Konzentratkammer sich sofort in der vorgelegten Flüssigkeitsmenge der Füllkammer verteilen können und der Lösungs-/Mischungsprozess begünstigt ist. Bevorzugt weist der Zuführport am Ende des Schlauchstücks Mittel zur Erzeugung einer Strömungsturbulenz im Inneren des Containers auf, die eine Durchmischung der Konzentrate mit dem Verdünnungsmittel begünstigen.

Weiterhin ist es vorteilhaft, dass der Container in der Ausgestaltung als Beutel aus elastischem, dehnbaren Folienmaterial hergestellt ist. Der Beutel vergrößert so sein Volumen mit zunehmender Befüllung durch das Verdünnungsmittel ähnlich einem Luftballon. Die Folienwandungen werden dabei elastisch gedehnt, so dass bei Entleerung des Beutels nach Gebrauch der medizinischen Lösung die Dehnung zurückgeht und der leere Beutel auf eine möglichst geringe Dimension zurückschrumpft. Dies liefert Handhabungsvorteile für das Pflegepersonal bei der Verwendung von großvolumigen Beuteln mit Füllvolumina von über 60 Litern. Weiterhin wirkt sich die Verwendung der dehnbaren Folie auf eine bessere Befüllung großvolumiger Beutel aus, da sich der Beutel beim Befüllen faltenfrei ausdehnt und weitgehend faltenfrei bei Entleerung schrumpft. Somit sind vollständige Befüllungen und Entleerungen möglich. Weiterhin setzt die dehnbare Folie den Kraftaufwand für den Anriss peelbarer Trennlinien herab. Ein eventuell entstehender zu hoher Kraftaufwand beim Lösen der Peelnaht wird durch Materialdehnung der Folie aufgenommen und wirkt somit materialschonend einem eventuellen Folienbruch entgegen. Eine bevorzugt zu verwendende Folie zum Aufbau eines erfindungsgemäßen Mehrkammerbeutels ist in der Deutschen Patentanmeldung DE 10 2010 014 785.0 beschrieben, auf deren Inhalt vollständig Bezug genommen wird. Darin wird eine elastisch dehnbare Mehrschichtfolie beschrieben, die mindestens eine an der Gesamtdicke der Folie bemessene relativ dünne äußere Schicht aufweist, die der Verschweißung dient, und mindestens eine an der Gesamtdicke der Folie bemessene relativ dicke mittlere Schicht aufweist, die im Wesentlichen für das mechanische Verhalten der Folie, insbesondere der elastischen Dehnbarkeit verantwortlich ist. Die äußere Schicht weist zwei thermoplastische Elastomere mit jeweils einem hohen und einem niedrigen Schmelzflussverhalten auf. Damit wird die Peelbarkeit der Peelnähte unter elastischer Dehnung der Folie optimiert, ohne dass die Peelnähte für die vorgesehene Beanspruchung zu schwach sind und ohne dass die Folie über die Dehnung zu stark beansprucht wird.

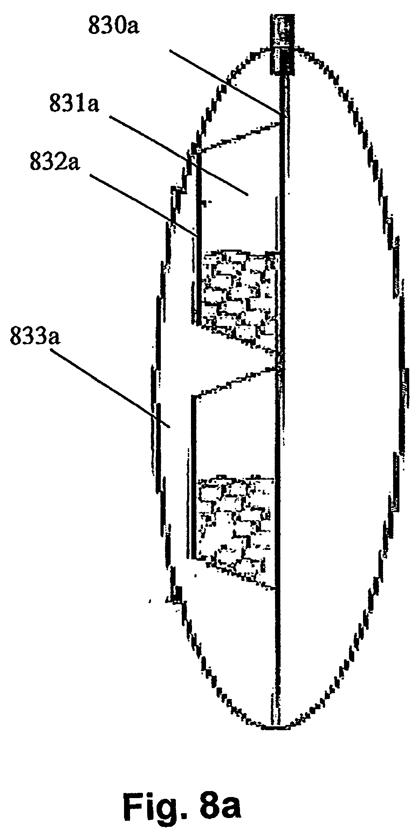

In einigen Fällen kann es sinnvoll sein Vorkehrungen zu treffen, die eine gleichmäßige Dehnung des elastisch dehnbaren Beutelmaterials während des Befüllvorgangs ermöglichen. Insbesondere, wenn die Konzentratkammern (Fig. 8a) des Beutels durch tief gezogene Folienwandabschnitte durch nur eine der beiden sich gegenüberliegenden Folien gebildet werden, kann eine ungleichmäßige Dehnung des Folienmaterials bei der Befüllung des Beutels eintreten. Dies kann dazu führen, dass die durch die Tiefziehung vorgeschwächte Folienseite stärker als die nicht vorgeschwächte Folienseite gedehnt wird. Der Beutel kann durch die Befüllung unter diesen Umständen Auswölbungen aufweisen, die zu verschiedenen Problemen führen können:
- partiell sehr hohe plastische Verformung der Folie und damit Gefahr der Rissbildung
- Einschränkungen von Funktionalitäten des Beutels, z.B. Verdeckung und Verschluss von Entlüftungsöffnungen und weiteren Zugansports
- Knicken der Anschlussschläuche

Das Problem der Schwächung von tief gezogenen Folienabschnitten eines Beutels während der Befüllung kann mit einem Beutel gelöst werden, der die folgenden Merkmale aufweist:
1) Symmetrisch expandierbarer Lösungsbeutel zu Aufnahme von Flüssigkeiten für die Nierenersatztherapie bestehend aus einer ersten Folie und einer zweiten Folie, die umfänglich durch eine permanente Begrenzungslinie verbunden sind und einen inneren Raum definieren, der durch Befüllung und Dehnung der Folien expandierbar ist, wobei die erste Folie zumindest teilweise für die Aufnahme von Konzentraten und die Bildung von Konzentratkammern tief gezogene Bereiche aufweist und die zweite Folie derart ausgebildet ist, dass bei Befüllung des umschlossenen Raums mit Flüssigkeit, die erste und die zweite Folie im wesentlichen gleichmäßig gedehnt werden.
2) Ein Beutel mit den Merkmalen nach Punkt 1), wobei die gleichmäßige Dehnung dadurch erreicht wird, dass die zweite Folie dünner als die erste Folie ausgebildet ist.
3) Ein Beutel mit den Merkmalen nach Punkt 1) oder 2), wobei die zweite Folie 2 - 20% dünner ausgebildet ist als die erste Folie.
4) Ein Beutel mit den Merkmalen nach den Punkten 1) bis 3), wobei die zweite Folie 5 bis 20 µm dünner ausgebildet ist als die erste Folie, insbesondere dann, wenn die erste Folie eine Dicke von 100µm bis 300µm aufweist.
5) Ein Beutel mit den Merkmalen nach Punkt 1), wobei die erste Folie und die zweite Folie gegenüberliegend tief gezogene Bereiche aufweist.

Für den Aufbau eines solchen Beutels können alle vorliegend beschriebenen Konstruktionsmerkmale herangezogen werden. Die umfängliche permanente Begrenzungslinie zwischen erster Folie und zweiter Folie kann aus einer Schweißnaht bestehen, wenn zwei einzelne Folien zu einem Beutel zusammengeschweißt werden. Wird ein Folienschlauch verwendet, wird die Begrenzungslinie nur abschnittsweise durch eine permanente Schweißnaht gebildet. In anderen Abschnitten wird die Begrenzungslinie dann durch die Folie selbst, bzw. in einer flachgelegten Form des Beutels durch eine Folienfalte gebildet.

Gegen eine asymmetrische Ausformung des Beutels während der Befüllung ist dafür Sorge zu tragen, dass die Folien durch den sich bildenden hydrostatischen Druck die gleiche Dehnung erfahren. Auf die Dehnung nehmen insbesondere die Folienstärke und das Elastizitätsmodul des Folienmaterials einen Einfluss.

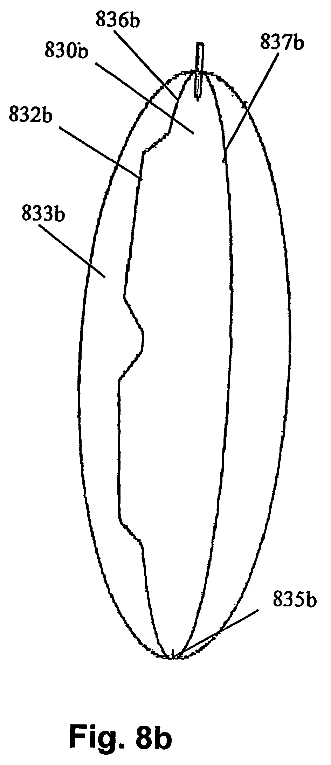

Für eine gleichmäßige Foliendehnung der ersten Folie, die tief gezogene Bereiche aufweist und der zweiten Folie, die keine tief gezogenenen Bereiche aufweist ist es in einer Ausführung daher vorteilhaft, wenn die zweite Folie dünner als die erste Folie ausgestaltet ist. Der Dickenunterschied der Folien ist dabei abhängig von der Folienstärke und der Materialeigenschaft (z.B. Elastizitätsmodul). Bei einer Foliendicke der ersten Folie von 190 µm kann daher vorgesehen sein die zweite Folie mit einer Dicke von 180 µm herzustellen, so dass sich bei Befüllung die Folien in gleichem Ausmaß dehnen. Die erste Folie weist die Foliendicke von 190 µm dabei in den nicht tief gezogenen Bereichen auf (Fig. 8b). Insbesondere kann eine gleichmäßige Dehnung von erster und zweiter Folie erreicht werden, wenn die zweite Folie 5 bis 30 µm dünner ausgebildet ist, als die erst Folie an den nicht tief gezogenen Bereichen. Dies ist insbesondere für Beutel mit einem Befüllungsvolumen von 5 bis 120 Litern zugrunde zu legen.

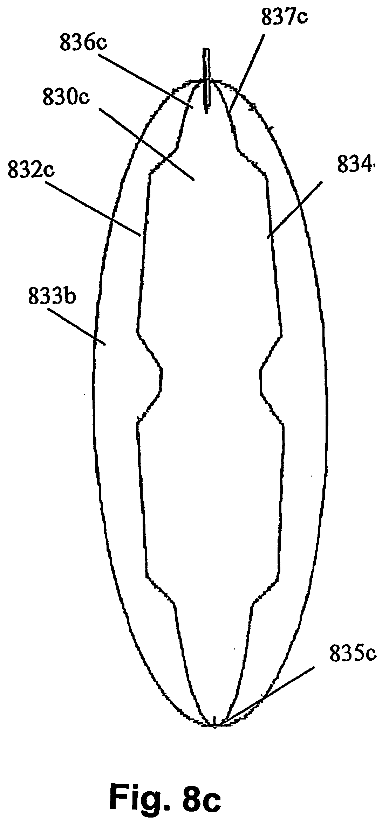

Alternativ kann vorgesehen sein, dass die zweite Folie und die erste Folie tief gezogene Bereiche aufweist. Die tief gezogenen Bereiche der ersten und der zweiten Folie liegen dabei gegenüber. Eine Dehnung der ersten Folie durch Befüllung des Beutels verursacht eine identische Dehnung der zweiten Folie, weil die Materialstärken der gegenüberliegenden Folienabschnitte stets gleich sind (Fig. 8c).

Der erfindungsgemäße Beutel ist unter anderem dafür vorgesehen, in einem System zur Herstellung von medizinischen Lösungen verwendet zu werden. Insbesondere handelt es sich bei dem System um eine Vorrichtung zu Herstellung und Bereitstellung einer gebrauchsfertigen Dialyselösung. Das System umfasst:
- den erfindungsgemäßen Mehrkammercontainer mit Konzentraten/Teillösungen zur Herstellung einer medizinischen Lösung, insbesondere Dialyselösungen,
- eine Kontrolleinheit zur Überwachung der Flüssigkeitsherstellung, vorzugsweise über Messung der elektrischen Leitfähigkeit.
- weitere geregelte und kontrollierte Pumpenmittel, die mit dem System zusammenwirken und ein Zuführen von Verdünnungsflüssigkeit zum Lösungscontainer des Vorlagensystems ermöglichen.
- weiterhin eine Stützstruktur, die dafür vorbereitet ist, einen erfindungsgemäßen Mehrkammercontainer oder Mehrkammerbeutel für die Herstellung der medizinischen Lösung aufzunehmen.

Zur Überwachung des Fortgangs des Lösungsprozess weist das System Mittel auf, um von einer entnommenen Probe den elektrischen Leitfähigkeitswert zu bestimmen. Es können aber auch Mittel vorhanden sein, um den Leitfähigkeitswert der Mischlösung während des Herstellprozesses kontinuierlich zu überwachen. In einer Ausführungsform kann ein Behälter oder ein Volumenabschnitt mit der im Verlauf der Herstellung der medizinischen Lösung anfallenden Mischlösung durchströmt werden und über Leitfähigkeitssensoren analysiert werden. Der Anstieg des Leitfähigkeitswertes während der Herstellung der Lösung wird durch die Leitfähigkeitssensoren detektiert und an eine Kontrolleinheit einer Prozessoreinheit übermittelt. Dabei kann in einer Speichereinheit ein Leitfähigkeitsprofil für die Herstellung der Lösung aus mehreren Teilkonzentraten/Teillösungen hinterlegt sein. Über eine Eingabeeinheit kann die Art des Lösungscontainers und die Konfiguration der Teilkonzentrate eingegeben und mit den hinterlegten Profilen verglichen werden. Stimmen im Verlauf der Herstellung der medizinischen Lösung in dem Lösungscontainer die zeitlichen Werte mit den Werten der entsprechenden in der Speichereinheit hinterlegten Werten überein, so wird die Lösung zur weiteren Verwendung von der Kontrolleinheit der Prozessoreinheit freigegeben. Andernfalls kann durch die Kontrolleinheit ein Signal verursacht werden, das anzeigt, dass die hergestellt Lösung nicht freigegeben ist.

Im Einklang mit dem Gegenstand der Erfindung steht weiterhin ein Verfahren zur Herstellung einer medizinischen Lösung aus mehreren Konzentraten mit einem zuvor beschriebenen Mehrkammercontainer. Gemäß dem Verfahren wird eine Flüssigkeit, zum Beispiel RO Wasser oder eine Teillösung in die eine Füllkammer (10, 210, 410, 510, 610, 710) des Containers (1, 201, 401,501, 601, 701) eingeleitet. Durch den sich aufbauenden hydrostatischen Druck der eingeleiteten Flüssigkeit wird während des Befüllungsvorgangs eine erste semipermanente Trennlinie (9, 209a, 209d, 409, 509, 609, 709a, 709c) aufgebrochen und das Konzentrat einer Konzentratkammer (3, 203a, 203b, 405, 503, 703a, 703b) freigesetzt und mit der Flüssigkeit der Füllkammer vermischt. Das freigesetzte Konzentrat oder das Konzentrat einer im weiteren Verlauf des Füllvorgangs aufgebrochenen Konzentratkammer (4, 204a, 204b, 404, 504, 704, 705) liefert im Wesentlichen keinen Beitrag zur elektrischen Leitfähigkeit der herzustellenden Lösung. Solche Komponenten können zum Beispiel organische Substanzen sein, die in wässrigen Systemen löslich sind, wie zum Beispiel Sacharide, Oligo- oder Polysacharide, polymere wasserlösliche Substanzen oder zum Beispiel Glukose, Fruktose, Maltodextrin, Icodextrin, Inuline usw.

Durch weiteres Befüllen wird anschließend die eine erste Trennlinie oder eine weitere semipermanente Trennlinie weiter aufgebrochen und das Konzentrat (C, C₁, C₂) einer weiteren Konzentratkammer (3, 203a, 203b, 503, 703a, 703b) freigesetzt, das einen Beitrag zur elektrischen Leitfähigkeit der Lösung liefert. Solche Substanzen können vor allem Salze sein, die in Mischung mit dem Verdünnungsmittel in dissoziierter Form vorliegen, zum Beispiel Natriumchloride, Magnesiumchloride, Kalziumchloride, Kaliumchloride, Natriumphosphate, Natriumcitrate, Natriumlaktate, Natriumcarbonate, Salze von schwachen Säuren, wie zum Beispiel Laktate, Acetate, Salze von Äpfelsäure, Fumarate, Oxalate, Succinate, Karbonate oder Bikarbonate, oder Salze von Aminosäuren.

Das Freisetzen eines Konzentrats mit Beitrag zur elektrischen Leitfähigkeit und eines Konzentrats ohne elektrische Leitfähigkeit kann zeitgleich erfolgen oder zeitlich versetzt, in jedem Fall aber so, dass das Konzentrat (A, B) ohne Beitrag oder ohne charakteristischen Beitrag zur elektrischen Leitfähigkeit in Lösung nicht nach dem Konzentrat mit Beitrag zur elektrischen Leitfähigkeit in Lösung (C, C₁, C₂) freigesetzt und gelöst wird. Durch dieses Verfahren wird sichergestellt, dass durch Leitfähigkeitsmessung das Lösen des Konzentrats mit Beitrag zur elektrischen Leitfähigkeit überwacht wird, wobei das Lösen des Konzentrats ohne Beitrag zur elektrischen Leitfähigkeit bereits erfolgt sein muss oder zeitgleich erfolgt. Mithilfe dieses Verfahrens unter Verwendung des angegebenen Containers kann das vollständige Lösen von Konzentraten durch Leitfähigkeitsmessungen erfolgen, wobei gewisse Konzentrate keinen Beitrag zur elektrischen Leitfähigkeit liefern. Vorzugsweise weist eine fertige medizinische Lösung zum Beispiel Dialyselösung eine spezifische elektrische Leitfähigkeit von 13,6 mS/cm auf. Je nach Zusammensetzung der gebrauchsfertigen Lösung ist im Sinne des erfindungsgemäßen Gegenstandes mit spezifischen Leitfähigkeitswerten von 10 bis 15 mS/cm zu rechnen. Konzentrate (A, B), die den spezifischen elektrischen Leitfähigkeitswert in Lösung in nur geringem Maße verändern, zum Beispiel um 0,5 mS/cm oder kleiner werden als unzureichend betrachtet, um deren Lösungsprozess durch Leitfähigkeitsmessungen überwachen zu können. Gerade bei der Herstellung von großvolumigen Vorräten der medizinischen Lösung, also zum Beispiel eines Dialyseflüssigkeitsvorrates von 60 bis 120 1, sind Fehlerbehaftungen des Herstellverfahrens nicht auszuschließen. Exaktere Messungen von elektrischen Leitfähigkeitswerten sind wohl technisch möglich, aber im Rahmen der Herstellung einer gebrauchsfertigen Lösung aus vorgelegten Konzentraten nicht einfach möglich, da zuviel Parameter den Lösungsprozess und damit die Leitfähigkeitsänderung beeinflussen. Leitfähigkeitsänderungen von 0,5 mS/cm und darunter, zum Beispiel 0,3 mS/cm und darunter oder 0,1 mS/cm und darunter können nicht als charakteristisch für eine Überwachung des Lösungsvorgangs im Sinne des erfindungsgemäßen Gegenstandes betrachtet werden. Konzentrate, die solch geringe Beiträge zur Leitfähigkeit der gebrauchsfertigen Lösung beitragen, werden im Sinne des erfindungsgemäßen Gegenstandes als Konzentrate bezeichnet, die keinen wesentlichen Beitrag zur elektrischen Leitfähigkeit einer herzustellenden Lösung beitragen.

Alternativ kann der Druck auch durch äußere Anwendung auf den Beutel erfolgen und die benötigte Kraft zur Lösung der semipermanenten Trennlinien aufbringen. Zur Überwachung der Herstellung der medizinischen Lösung dient auch hier vorzugsweise eine elektrische Leitfähigkeitsüberwachung.

In einer bevorzugten Ausführung wird als Container zur Anwendung des erfindungsgemäßen Verfahrens ein Beutel benutzt. Insbesondere kann der Beutel ein großvolumiger Beutel sein, worunter ein Volumen von 5-1201 zu verstehen ist.

Die Verwendung eines Beutels für das erfindungsgemäße Verfahren hat den Vorteil, dass die begrenzenden Trennlinien durch lösbare Peelnähte ausgestaltet sein können. Peelnähte sind produktionstechnisch einfach durch Kunststoffschweißwerkzeuge herstellbar, indem bestimmte, dem Folienmaterial entsprechende Siegeltemperaturen vorgegeben werden, so dass keine permanente Schweißung der Folienstücke stattfindet. Die erforderlichen Peelnahtstärken sind mit den beschriebenen Peelnahtstärken des Mehrkammerbeutels identisch.

Durch unterschiedliche Ausgestaltung der Peelnahtfestigkeiten kann das sequentielle Aufbrechen der Konzentratkammer während des Befüllvorgangs beeinflusst werden. So kann zum Beispiel ein Peelnahtabschnitt, der eine Konzentratkammer mit Konzentraten ohne Beitrag zur elektrischen Leitfähigkeit gegenüber der Füllkammer begrenzt, schwächere Peelnahtfestigkeiten besitzen. Ein Peelnahtabschnitt, der eine Konzentratkammer mit Konzentraten, die einen Beitrag zur elektrischen Leitfähigkeit aufweisen, gegenüber der Füllkammer trennt, kann eine stärkere Peelnahtfestigkeit aufweisen. Das Konzentrat ohne Beitrag zur elektrischen Leitfähigkeit wird dabei mit geringerer Kraft freigesetzt, als das Konzentrat mit Beitrag zur elektrischen Leitfähigkeit. Damit wird das im Füllverfahren bestehende Erfordernis erfüllt, dass Konzentrate ohne Beitrag zur elektrischen Leitfähigkeit vor oder gleichzeitig mit Konzentraten mit Beitrag zur elektrischen Leitfähigkeit gelöst werden.

Bevorzugt sind Verfahren, bei denen der Druck im Inneren des Beutels durch Zuführen des Verdünnungsmittels in das Beutelinnere wirkt. In diesem Fall wird das Verdünnungsmittel, zum Beispiel Wasser, durch eine Quelle, zum Beispiel einer RO (Reverse Osmosis) Anlage, bereitgestellt. Der Beutel kann in einer Ausgestaltung mit trockenen Konzentraten mit relativ schwachen Peelnahtfestigkeiten der Trennlinien hergestellt werden. Dies ist vorteilhaft, da so die aufzuwendenden Drücke zur Lösung der Trennlinien gering gehalten werden können.

Entsprechend können auch Materialfestigkeiten der für den Beutel verwendeten Folie und die Foliendicken geringer gewählt werden, wodurch Material. und Herstellkosten gespart werden.

In einer Ausführung ist vorgesehen, dass ein externes Pumpmittel Flüssigkeit zur Verfügung stellt, die durch einen Port in das Innere des Beutels geleitet wird. Der sich aufbauende innere Fülldruck wird bei dem erfindungsgemäßen Verfahren über die externe Pumpe durch den Zugangsport aufgebaut. Bei zunehmendem Flüssigkeitsdruck im Inneren des Beutels werden die Trennlinien geöffnet, die Kammerinhalte freigesetzt und mit dem zuströmenden Verdünnungsmittel vermischt.

Die Anordnung des Ports an dem Beutel und das Zuströmen des Verdünnungsmittels erfolgen dabei so, dass eine erste Kammer geöffnet wird. Vorzugsweise umfasst der Kammerinhalt der einen ersten Kammer Komponenten, die keinen Beitrag zur elektrischen Leitfähigkeit der Mischlösung aufweisen. Insbesondere kommen hierbei organische Verbindungen, wie zum Beispiel Glukose, Sorbitol, Fruktose, osmotisch wirksame Substanzen wie wasserlösliche Polymere, zum Beispiel Polyglukosen, Polyfruktosen, Polyether infrage.

Im weiteren Verlauf der Beutelfüllung wird eine weitere Kammer aufgebrochen und der Kammerinhalt mit dem Verdünnungsmittel vermischt. Bei den Kammerinhalten handelt es um Komponenten, die in Lösung einen Beitrag zur elektrischen Leitfähigkeit der Lösung beitragen. Bei Dialyselösungen handelt es sich hier insbesondere um wasserlösliche Salze, zum Beispiel Natriumchlorid, Magnesiumchlorid, Kalziumchlorid, Natriumphosphate, Kaliumchlorid, Salze von schwachen Säuren, zum Beispiel Natriumlaktat, Natriumacetat, Natriumbikarbonat und oder Natriumcarbonat oder auch Natriumcitrat. Der Leitfähigkeitssensor stellt im Zuge der Auflösung der Komponenten dieser Kammer eine Zunahme der Leitfähigkeit fest. Da nach der Beutelkonstruktion die eine erste Kammer mit Komponenten, die keinen Beitrag zur elektrischen Leitfähigkeit liefern, im Zuge, also vor oder gleichzeitig, mit der einen zweiten Kammer, die einen Beitrag zur elektrischen Leitfähigkeit der Lösung liefern, geöffnet wird, ist mit der Leitfähigkeitsänderung durch Auflösen der einen zweiten Kammer auch die Öffnung der einen ersten Kammer sicher gestellt.

### Detaillierte Beschreibung der Erfindung anhand der Zeichnungen

Die folgenden Beispiele sind Ausführungen, die im Einklang mit dem Gegenstand der Erfindung stehen, ohne dass der Gegenstand der Erfindung durch diese Beispiele in einer einschränkenden Art und Weise zu verstehen ist. Dem Fachmann erschließt sich aus dem Kontext der Beschreibung, dass die Erfindung auch über diese beispielhaften Ausführungsformen hinaus anwendbar ist.

In einer Ausführung des erfindungsgemäßen Containers zur Verwendung in der Dialyse werden vorzugsweise mehrere Konzentratteile verwendet, die in mehreren Kammern des Beutels gelagert werden. Vorzugsweise handelt es sich bei den Konzentraten um Festkonzentrate, wobei flüssige Konzentrate oder andere Konzentratformen mit dem erfinderischen Beutel gleichermaßen verwendet werden können.

Eine bevorzugte Konzentratzusammensetzung in einem erfindungsgemäßen Mehrkammerbeutel für die Herstellung einer gebrauchsfertigen Dialyselösung ergibt sich aus folgender Tabelle. Die Bereitstellung mehrerer Konzentrate für die Herstellung einer Dialyselösung ist Gegenstand der DE 102010039489.0, auf die hiermit vollständig Bezug genommen wird:

**Tabelle 1**

| Komponente | Konzentratkammer | Einwaage [g] | Konzentration in gebrauchfertiger Lösung [mmol/l] | Beitrag zur elektrischen Leitfähigkeit in Lösung |
|---|---|---|---|---|
| Basisches Magnesiumcarbonat 4MgCO₃ x Mg(OH)₂ x 5H₂O | A | 3,01 | 0,5 | Im Wesentlichen nicht vorhanden |
| D-Glukose wasserfrei | B | 62 | 5,55 | Im Wesentlichen nicht vorhanden |
| Calciumchlorid wasserfrei | A | 8,62 | 1,25 | Im Wesentlichen nicht vorhanden |
| Zitronensäure | A | 11,97 | 1,0 | Im Wesentlichen nicht vorhanden |
| Kochsalz | C | 391,22 | 140 | Vorhanden |
| Natriumhydrogencarbonat | C | 166,78 | 32 | Vorhanden |
| Kaliumchlorid | A | 28,1 | 2 | Im Wesentlichen nicht vorhanden |

Dabei werden ein oder mehrere Lösungskomponenten auf drei (A, B, C) Konzentratkammern aufgeteilt. Demnach wird:
- Glukose ohne weitere Substanzen in Kompartiment B gelagert
- Die Salze Magnesiumcarbonat, Calciumchlorid, Natriumchlorid, Kaliumchlorid und Zitronensäure werden in einem weiteren Kompartiment A gelagert.
- Natriumchlorid und Natriumbikarbonat (optional Kaliumhydrogencarbonat) werden in einem weiteren Kompartiment C gelagert

Die Komponenten, die bevorzugt in Konzentrat A zusammengefasst sind, können physikalisch eine elektrische Leitfähigkeitsänderung in Lösung bewirken, da es sich in Lösung um dissoziierte Salze und somit um Ladungsträger handelt. Jedoch sind diese Substanzen in einer Dialyselösung, zwar essentiell, aber für eine Überwachung der Herstellung der Dialyselösung durch Leitfähigkeitsmessung aus den entsprechenden Konzentraten in zu geringer Menge vorhanden, als dass sich aus den Messwerten ein verlässliches Ergebnis über den Lösungszustand des entsprechenden Konzentrats ableiten ließe. Somit muss auch dieses Konzentrat A für die Herstellung einer Dialyselösung als ein Konzentrat angesehen werden, das im Wesentlichen keinen Beitrag zur elektrischen Leitfähigkeit der fertigen Dialyselösung beiträgt.

Die Aufteilung der Komponenten in drei Konzentratteile erfolgt aus Gründen der Lagerstabilität. Generell ist darauf zu achten, dass keine Degradierung von Konzentraten während der Lagerung stattfindet. Es hat sich gezeigt, dass feste Konzentrate von Natriumbikarbonat, Zitronensäure und Glukose in gemeinsamer Lagerung inakzeptable Veränderungen eingehen. Es finden dabei Reaktionen des Glukoseabbaus statt. Die trockenen Konzentrate neigen in Mischung zur Wasseraufnahme durch Luftfeuchtigkeit, so dass Agglomerate entstehen, die nicht schnell genug gelöst werden können. Daher sind Bikarbonatsalze getrennt von Glukose und getrennt von Zitronensäure zu lagern. Glukose ist ebenfalls getrennt von Zitronensäure zu lagern. Ebenso ist zur Lagerung Natriumchlorid von Glukose zu trennen. Die Aufteilung der Konzentrate resultiert in einer mindestens dreiteiligen Lagerung der Konzentrate, wobei Glukose isoliert gelagert werden muss und damit ein Teilkonzentrat darstellt, das in Lösung keinen Beitrag zur elektrischen Leitfähigkeit aufweist. Weitere bevorzugte Ausführungen werden anhand der Figuren erläutert.

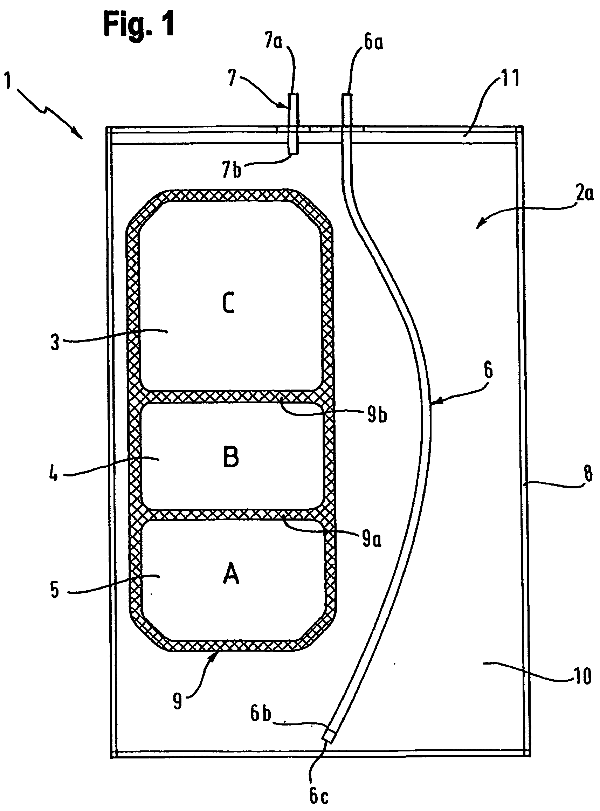

**Figur 1** zeigt einen Container 1 mit einer umfänglichen permanenten Begrenzungslinie 8. Bevorzugt handelt es sich bei dem Container um einen Beutel und die umfängliche Begrenzungslinie ist aus einer permanenten Schweißnaht gebildet. Der Beutel setzt sich zusammen aus einer, aus Perspektive der Figur 1 gesehenen, oberen Außenwandung 2a, die bevorzugt aus einem Folienblatt hergestellt ist. Weiterhin wird der Container oder Beutel durch eine weitere nicht gezeigte, aus Perspektive der Figur 1 gesehene, untere Außenwandung 2b gebildet. Der Beutel verfügt weiterhin über eine Konzentratkammer A mit einem Konzentrat 5, eine weitere Konzentratkammer mit Konzentrat 4, eine weitere Konzentratkammer C mit Konzentrat 3. Die Konzentratkammer wird durch eine erste Trennlinie 9 umschlossen, die wenigstens teilweise semipermanent ist. Die erste Konzentratkammer A ist durch eine zweite, wenigstens abschnittsweise semipermanente Trennlinie 9a von der weiteren Konzentratkammer B getrennt. Konzentratkammer B ist von der weiteren Konzentratkammer C durch eine, wenigstens abschnittsweise semipermanente dritte Trennlinie 9b getrennt. Vorzugsweise sind alle Trennlinien 9, 9a, 9b, wenigstens abschnittsweise semipermanent ausgestaltet und in einer besonders bevorzugten Ausführung sind alle Trennlinien 9, 9a, 9b vollständig semipermanent. Weiterhin ist es von Vorteil, wenn die Trennlinien durch Peelnähte hergestellt sind.

Der Beutel beinhaltet weiterhin einen Port 7 mit einem ersten Ende 7a außerhalb des und einem zweiten Ende im Inneren des Beutels. Ein weiterer Port 6 verbindet das Innere des Beutels über ein erstes Ende im Inneren des Beutels 6b mit einem weiteren Ende 6a außerhalb des Beutels. Der Port 6 ist dafür vorgesehen, mit weiteren Fluidfördermitteln, zum Beispiel einer Pumpe zu kommunizieren. Die Ports 7 und 6 sind vorzugsweise durch Schweißverbindungen fluiddicht in der umfänglichen Umfassungslinie 8 befestigt. Vorzugsweise befindet sich am Ende 6b des Ports 6 ein Mittel 6c zur Erzeugung von Fluidturbulenzen des zuströmenden Verdünnungsmittels. Dieses Mittel kann als Turbulenz erzeugende Düse ausgestaltet sein oder als eine Turbulenz erzeugende Fritte.

Weiterhin besteht Port 6 aus einem Schlauch, der den Beutel im Inneren in seiner länglichen Ausdehnung durchmisst. Es wird so im Befüllverfahren gewährleistet, dass zum Beispiel bei aufrechter Lagerung durch Aufnahme des Beutels an einer integralen Halteschiene 11 der Beutel von unten befüllt wird und die Konzentratkammern A, B, C in der Abfolge A, B, C durch den inneren Fülldruck geöffnet werden.

In einer Ausführungsform beinhaltet Konzentratkammer A ein Konzentrat 5, das aus Calciumchlorid, Natriumchlorid, Kaliumchlorid und Zitronensäure besteht und einen geringen Beitrag zur elektrischen Leitfähigkeit in Lösung aufweist. Konzentratkammer B beinhaltet ein Glukosekonzentrat 4, das keinen Beitrag zur elektrischen Leitfähigkeit in Lösung aufweist. Konzentratkammer C beinhaltet ein Konzentrat 3, bestehend aus Natriumchlorid und Natriumbikarbonat optional Kaliumhydrogencarbonat, das einen Beitrag zur elektrischen Leitfähigkeit in Lösung aufweist.

Der Beutel weist weiterhin einen inneren Raum 10 auf, der eine Füllkammer darstellt und bevorzugt leer ist. In der bevorzugten Beutelversion des Containers wird durch Zuströmen des Verdünnungsmittels in die Füllkammer 10 die gegenüberliegenden Folienblätter 2a, 2b auseinander gedrückt, so dass bei weiterer Befüllung eine Zugspannung auf die Trennlinien 9, 9a, 9b ausgeübt wird und diese gelöst werden, so dass sich die Teilkonzentrate 5,4,3 mit dem Verdünnungsmittel vermischen.

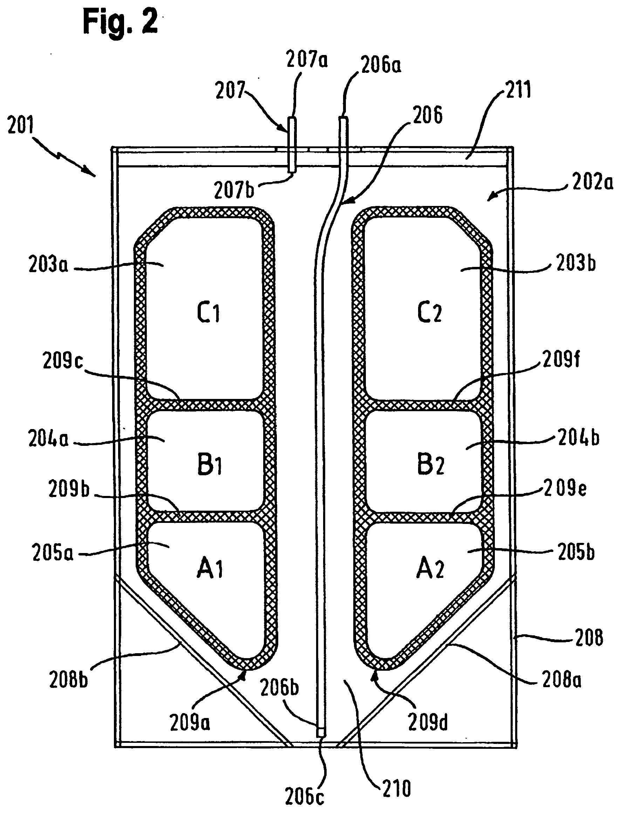

In **Figur 2** ist eine weitere Darstellung eines Containers 201, vorzugsweise eines Beutels, dargestellt. Der Beutel setzt sich zusammen aus einem, aus Perspektive der Figur 2 gesehenen, oberen Folienblatt 202a und einem nicht gezeigten unteren Folienblatt 202b. Der Beutel verfügt weiterhin über eine Konzentratkammer A1 mit Konzentrat 205a. Nach den Ausführungen der Tabelle 1 befinden sich in der Konzentratkammer A1 die Substanzen Magnesiumcarbonat oder allgemein lösliche Magnesiumsalze, Calciumchlorid oder allgemein lösliche Kalziumsalze, Zitronesäure oder Zitratsalze. Das Konzentrat 205a liefert einen nur geringen Beitrag zur elektrischen Leitfähigkeit in Lösung.

Eine weitere Konzentratkammer B1 enthält ein Konzentrat 204a. In einer beispielhaften Ausführung besteht das Konzentrat 204a aus einem Glukosekonzentrat, das keinen Beitrag zur elektrischen Leitfähigkeit in Lösung beiträgt.

Eine weitere Konzentratkammer C1 mit einem Konzentrat 203a enthält weitere Substanzen, die mit den Konzentraten 205a, 204a unverträglich sind, also zur Degradation neigen oder unerwünschte Wechselwirkung eingehen. Das Konzentrat 203a weist in Lösung einen Beitrag zur elektrischen Leitfähigkeit auf. Insbesondere handelt es sich bei dem Konzentrat 203a um Natriumchlorid oder allgemein um Natriumsalze und Natriumbikarbonat oder allgemein um Salze der Kohlensäure.

Die Kammern werden durch eine geschlossene Umfassungslinie umschlossen, die aus einer Trennlinie 209a gebildet ist, die wenigstens teilweise semipermanente Abschnitte aufweist. Vorzugsweise besteht die Trennlinie 209a aus einer Peelnaht. Die Inhalte der Konzentratkammern A1, B1, C1 werden durch die weiteren Trennlinien 209b, 209c voneinander getrennt. Die weiteren Trennlinien 209b, 209c können permanente Schweißlinien, teilweise semipermanente Schweißlinien oder Peelnähte darstellen. In einer bevorzugten Ausführungsform bilden die Peelnähte 209a, 209b, 209c eine integrale Konstruktion aus Peelnahtabschnitten, die ineinander übergehen und vollständig lösbar sind.

Die beispielhafte Ausführung aus Fig. 2 ist weiterhin dadurch gekennzeichnet, dass ein Port 206 das Innere des Beutels 210 über ein erstes Ende im Inneren des Beutels 206b und einem weiteren Ende außerhalb des Beutels 201 verbindet. Port 206 ist vorzugsweise durch Schweißverbindungen fluiddicht in der umfänglichen Umfassungslinie 208 befestigt. Vorzugsweise befindet sich am Ende 206b des Ports 206 ein Mittel 206c zur Erzeugung von Fluidturbulenzen von zuströmenden Verdünnungsmittel. Dieses Mittel kann als Turbulenz erzeugende Düse ausgestaltet sein oder als eine Turbulenz erzeugende Fritte. Weiterhin besteht Port 206 aus einem Schlauch, der den Beutel im Inneren 210 in seiner länglichen Ausdehnung durchmisst. Es wird so im Befüllverfahren gewährleistet, dass zum Beispiel bei aufrechter Lagerung durch Aufnahme des Beutels an einer integralen Halteschiene 211 der Beutel von unten befüllt wird und die Konzentratkammern A1, B1, C1 in der Abfolge A, B, C durch den inneren Fülldruck geöffnet werden.

Ein weiterer Port 207 mit einem ersten Ende 207a außerhalb des Beutels und einem weiteren Ende 207b innerhalb des Beutels dient zur Entnahme oder zur Zuführung von Lösung, Substanzen, Medikamenten etc.

Weiterhin zeigt die Ausführung in Fig. 2 einen zweiten Satz von Kompartimenten A2, B2, C2, die durch eine weitere Trennlinie entlang einer zweiten geschlossenen Umfassungslinie 209d umschlossen sind. Weitere Trennlinien 209e und 209f trennen die Inhalte der Kammern A2, B2, C2. In einer bevorzugten Ausführungsform bilden die Peelnähte 209d, 209e, 209f eine integrale Konstruktion aus Peelnahtabschnitten, die ineinander übergehen und vollständig lösbar sind.

In der beispielhaften Ausführungsform besteht der Inhalt der Konzentratkammer A2 aus einem Konzentrat 205b, das einen geringen Beitrag zur elektrischen Leitfähigkeit in Lösung aufweist. Das Konzentrat 205b in Konzentratkammer A2 kann mit dem Konzentrat 205a identisch sein oder nur einen Teil der Komponenten von Konzentrat 205a enthalten. In einer beispielhaften Ausführung bestehen die Lösungskomponenten 205b aus Magnesiumcarbonat oder allgemein aus löslichen Magnesiumsalzen, Calciumchlorid oder allgemein aus löslichen Kalziumsalzen, Zitronesäure oder Zitratsalzen oder einer anderen Säure.

Eine weitere Konzentratkammer B2 mit Lösungskomponenten 204b enthält in einer beispielhaften Ausführung die Substanzen, die keinen Beitrag zur elektrischen Leitfähigkeit in Lösung beitragen. In einer beispielhaften Ausführung kann es sich dabei um wasserfreie Glukose handeln. Das Konzentrat 204b kann mit 204a identisch sein oder weitere Substanzen enthalten, die keinen Beitrag zur elektrischen Leitfähigkeit liefern.

Eine weitere Konzentratkammer C2 mit Konzentrat 203b enthält weitere Substanzen, die mit den Substanzen der Lösungskomponenten 205b, 204b unverträglich sind, also zur Degradation neigen oder unerwünschte Wechselwirkung eingehen. Darüber hinaus weist dieses Konzentrat in Lösung einen Beitrag zur elektrischen Leitfähigkeit auf. Wird Konzentratkammer C2 aufgebrochen und eine Zunahme der elektrischen Leitfähigkeit durch Lösen des Konzentrats 203b festgestellt, so ist sicher auch das Konzentrat 204b gelöst worden. Die Substanzen der Lösungskomponenten 204b können mit den Substanzen 204a identisch sein oder nur einen Teil oder weitere Substanzen 204a enthalten. Insbesondere besteht das Konzentrat aus Natriumchlorid oder allgemein aus Natriumsalzen, Natriumbikarbonat oder allgemein aus Salzen der Kohlensäure.

Die Ausführungsform zeigt weiterhin eine Umfassungslinie 208, die aus einer permanenten Schweißnaht besteht. Zusätzliche permanente Schweißlinien Abschnitte 208a und 208b begrenzen den Containerinhalt oder Beutelinhalt so, dass ein schräger Beutelboden entsteht. Die Konstruktion begünstigt die Verwirbelung des zuströmenden Verdünnungsmittels durch Fluidturbulenz erzeugende Mittel 206c und damit den Lösungsprozess der Komponenten 205a,205b,204a,204b,203a,203b.

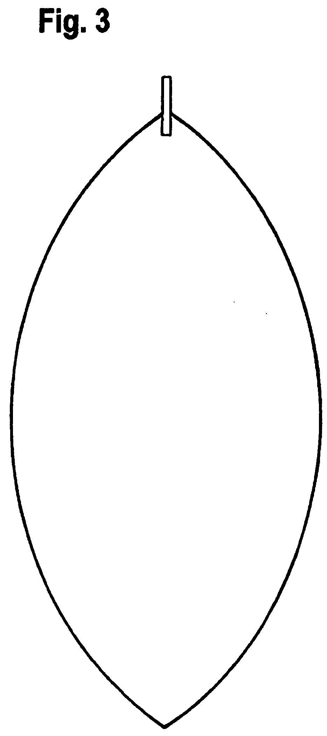

**Figur 3** zeigt einen seitlichen Querschnitt einer Ausführungsform im gefüllten Zustand. Insbesondere stellt diese Ansicht eine Ausführung dar, in der alle Trennlinien semipermanent und bereits durch die Befüllung gelöst worden sind.

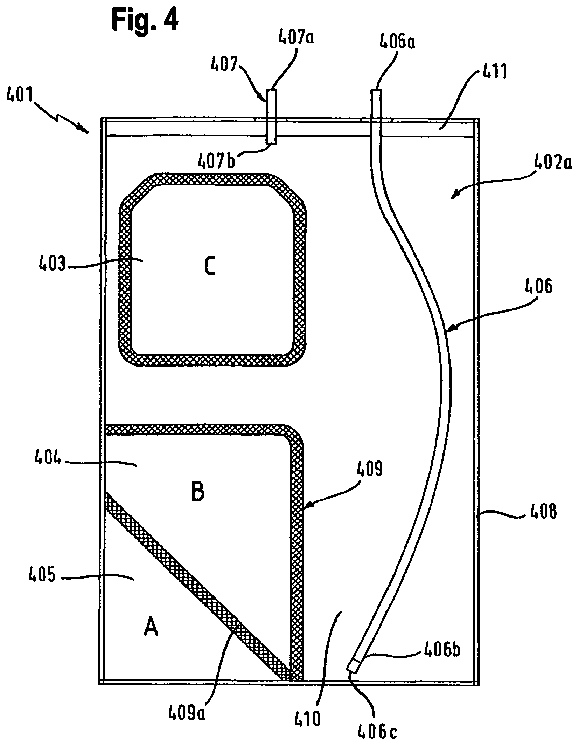

**Figur 4** zeigt eine alternative, nicht beanspruchte Ausführungsform 401. Der Container, vorzugsweise Beutel, weist eine, aus einer Perspektive aus Figur 4 gesehenen, oberen Außenwandung 402a und eine untere, nicht gezeigte Außenwandung 402b auf, die in einer bevorzugten Version die oberen und unteren Folienblätter eines Beutels sind. Der Beutel besteht weiterhin aus den Konzentratkammern A, B, C mit den Konzentraten 405, 404, 403. Dabei kann das Konzentrat 405 in Kammer A nach Tabelle 1 mit den Konzentraten 205a, 205b, 5 der vorangehenden Ausführungsformen 1, 201 identisch sein und einen geringen Beitrag zur elektrischen Leitfähigkeit in Lösung liefern.
Das Konzentrat 404 in Konzentratkammer B kann nach Tabelle 1 mit dem Konzentrat 204a, 204b, 4 der vorangehenden Ausführungsformen 1, 201 identisch sein und keinen Beitrag zur elektrischen Leitfähigkeit der Lösung liefern.
Das Konzentrat 403 in Konzentratkammer C kann nach Tabelle 1 mit den Konzentraten 3, 203a, 203b der vorangehenden Ausführungsformen 1, 201 identisch sein und einen Beitrag zur elektrischen Leitfähigkeit der Lösung liefern.

Die Konzentratkammern A und B werden durch eine Umfassungslinie 409 umschlossen, die in Abschnitten aus einem Teil der Umfassungslinie 408 und zum weiteren Teil aus einer Trennlinie gebildet ist, die wenigstens teilweise semipermanent ist. Vorzugsweise besteht die erste Trennlinie der Umfassungslinie 409 aus einer Peelnaht und bildet in einem Abschnitt 412 eine Begrenzung der Konzentratkammern A und B. Die Inhalte der Konzentratkammern A, B, werden durch die zweite Trennlinie 409a voneinander getrennt.

Die zweite Trennlinie 409a ist wenigstens abschnittsweise semipermanent und bevorzugt eine vollständig semipermanente Schweißlinie, wie zum Beispiel eine Peelnaht. In der einen dargestellten bevorzugten Ausführungsform bilden die Peelnähte der Trennlinien aus 409 und 409a eine integrale Konstruktion aus Peelnahtabschnitten, die ineinander übergehen, so dass sich ein beginnender Peelnahtbruch der Trennlinie aus 409 auch auf die Trennlinie 409a fortsetzt.

Ein Port 406 verbindet das Innere 410 des Beutels über ein erstes Ende im Inneren des Beutels 406b und einem weiteren Ende 406a außerhalb des Beutels 401. Port 406 ist vorzugsweise durch Schweißverbindungen fluiddicht in der umfänglichen Trennlinie 408 befestigt. Vorzugsweise befindet sich am Ende 406b des Ports 406 ein Mittel 406c zur Erzeugung von Fluidturbulenzen des zuströmenden Verdünnungsmittels. Dieses Mittel kann als Turbulenz erzeugende Düse ausgestaltet sein oder als eine Turbulenz erzeugende Fritte. Weiterhin besteht Port 406 aus einem Schlauch, der den Beutel im Inneren in seiner länglichen Ausdehnung durchmisst. Es wird so im Befüllverfahren gewährleistet, dass zum Beispiel bei aufrechter Lagerung durch Aufnahme des Beutels an einer integralen Halteschiene 411 der Beutel von unten befüllt wird und die Konzentratkammern A, B, C in der Abfolge A, B, C durch den inneren Fülldruck geöffnet werden.

Ein weiterer Port 407 mit einem ersten Ende 407a außerhalb des Beutels und einem weiteren Ende 407b innerhalb des Beutels dient zur Entnahme oder zur Zuführung von Lösung, Substanzen, Medikamenten etc.

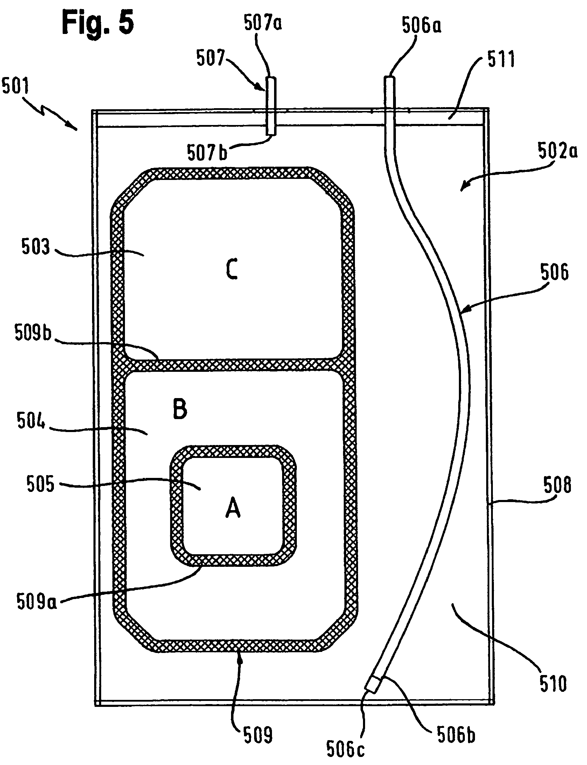

**Figur 5** zeigt eine weitere alternative Ausführung. Der Container, vorzugsweise Beutel, weist in Perspektive der Figur 5 eine obere Außenwandung 502a und eine untere nicht gezeigte Außenwandung 502b auf, die in einer bevorzugten Version die oberen und unteren Folienblätter eines Beutels sind. Der Beutel besteht weiterhin aus den Konzentratkammern A, B, C mit den Konzentraten 505, 504, 503. Dabei kann das Konzentrat 505 in Konzentratkammer A nach Tabelle 1 mit den Konzentraten 5, 205a, 205b der vorangehenden Ausführungsformen 1, 201 identisch sein und einen geringen Beitrag zur elektrischen Leitfähigkeit der Lösung liefern.

Das Konzentrat 504 in Konzentratkammer B kann nach Tabelle 1 mit den Konzentraten 4, 204a, 204b der vorangehenden Ausführungsformen 1, 201 identisch sein und keinen Beitrag zur elektrischen Leitfähigkeit der Lösung liefern.

Das Konzentrat 503 in Konzentratkammer C kann nach Tabelle 1 mit den Konzentraten 3, 203a, 203b vorangehenden Ausführungsformen 1, 201 identisch sein und einen Beitrag zur elektrischen Leitfähigkeit der Lösung liefern.

Die Konzentratkammern A und B sind entlang einer geschlossenen Umfassungslinie 509 durch eine wenigstens abschnittsweise semipermanente Trennlinie. Vorzugsweise ist die Trennlinie aus einer durchgehenden Peelnaht gebildet. Die Inhalte der Konzentratkammern A, B, C werden durch die weiteren Trennlinien 509a, 509b voneinander getrennt. Eine erste Trennlinie 509 begrenzt die Konzentratkammern B und C und umschließt die Kammer A. Eine zweite Trennlinie 509a trennt die Konzentratkammer A und B. Eine weitere Trennlinie 509b trennt die Konzentratkammer B und C. Die Trennlinie 409a ist wenigstens abschnittsweise semipermanent und bevorzugt eine vollständig semipermanente Schweißlinie, wie zum Beispiel eine Peelnaht. Ebenso besteht die Trennlinie 509b vorzugsweise aus einer Peelnaht. In der einen dargestellten bevorzugten Ausführungsform bilden die Peelnähte der Trennlinie aus 509 und 509b eine integrale Konstruktion aus Peelnahtabschnitten, die ineinander übergehen, so dass sich ein beginnender Peelnahtbruch der Trennlinie aus 509 auch auf die Trennlinie 509b fortsetzt.

Ein Port 506 verbindet das Innere des Beutels über ein erstes Ende im Inneren des Beutels 506b und einem weiteren Ende 506a außerhalb des Beutels 501. Port 506 ist vorzugsweise durch Schweißverbindungen fluiddicht in der umfänglichen Trennlinie 508 befestigt. Vorzugsweise befindet sich am Ende 506b des Ports 506 ein Mittel 506c zur Erzeugung von Fluidturbulenzen des zuströmenden Verdünnungsmittel. Dieses Mittel kann als Turbulenz erzeugende Düse ausgestaltet sein oder als eine Turbulenz erzeugende Fritte. Vorzugsweise besteht Port 506 aus einem Schlauch, der den Beutel im Inneren in seiner länglichen Ausdehnung durchmisst. Es wird so im Befüllverfahren gewährleistet, dass zum Beispiel bei aufrechter Lagerung durch Aufnahme des Beutels an einer integralen Halteschiene 511 der Beutel von unten befüllt wird und die Konzentratkammern A, B, C in der Abfolge B, A, C durch den inneren Fülldruck geöffnet werden.

Durch Befüllen des Beutels 501 durch den Port 506 mit Verdünnungsmittel strömt das Verdünnungsmittel in die Füllkammer 510. Bei zunehmendem Füllgrad wirkt auf die Trennlinie 509 eine Zugspannung, die dazu führt, dass die Trennlinie 509 zum Teil gelöst und das Konzentrat 504 mit dem Verdünnungsmittel vermischt wird. Bei weiterer Befüllung öffnet sich auch die Konzentratkammer A durch Lösen der Trennlinie 509a und das Konzentrat 505 vermischt sich mit dem Verdünnungsmittel und bereits angelöstem oder gelöstem Konzentrat 504. Dabei liefert Konzentrat 505 einen Beitrag zur elektrischen Leitfähigkeit in Lösung. Durch weiteres Befüllen des Beutels 501 löst sich die Trennlinie 509 und die Trennlinie 509b vollständig, so dass die Konzentrate mit dem Verdünnungsmittel vermischt werden. Die Mengen an Lösungskomponenten und Verdünnungsmitteln sind so bemessen, dass eine fertige physiologisch annehmbare Lösung, insbesondere Dialyselösung entsteht. Durch Leitfähigkeitsänderungen, die im Herstellprozess der medizinischen Lösung auf Lösen oder Verdünnen der Konzentrate in Konzentratkammer A zurückzuführen sind, kann sicher geschlossen werden, dass die Umfassungslinie 509 aufgebrochen wurde und die Kammerinhalte B, die nicht zur Leitfähigkeit der herzustellenden Lösung beitragen, freigesetzt und gelöst wurden.

Ein weiterer Port 507 mit einem ersten Ende 507a außerhalb des Beutels und einem weiteren Ende 507b innerhalb des Beutels dient zur Entnahme oder zur Zuführung von Lösung, Substanzen, Medikamenten etc.

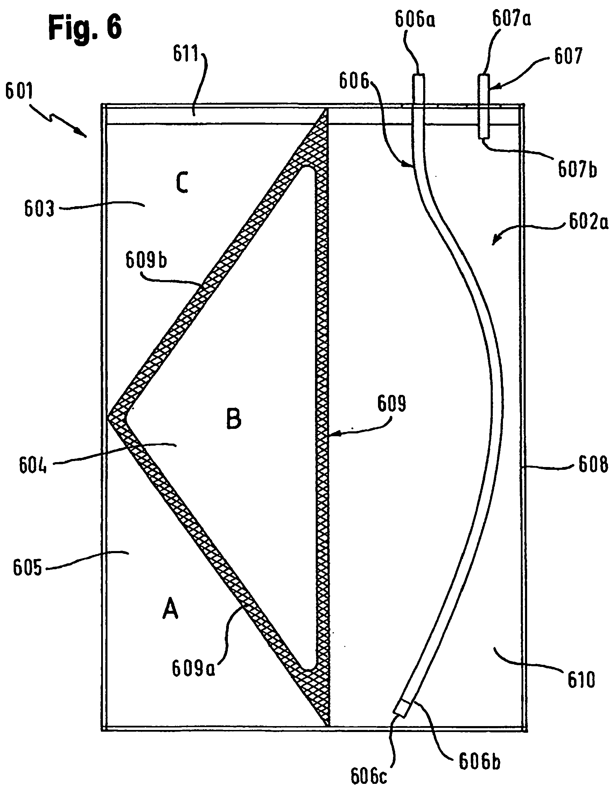

**Figur 6** zeigt eine weitere nicht beanspruchte Ausführungsform. Der Container 601, vorzugsweise Beutel, weist in Perspektive der Figur 6 eine obere Außenwandung 602a und eine untere nicht gezeigte Außenwandung 602b auf, die durch einen gemeinsamen umfänglichen Rand 608 begrenzt sind und fluiddicht verbunden sind. In einer bevorzugten Version sind 602a und 602b die oberen und unteren Folienblätter eines Beutels. Der Beutel besteht weiterhin aus den Konzentratkammern A, B, C mit den Konzentraten 605, 604, 603. Dabei kann das Konzentrat 605 in Konzentratkammer A nach Tabelle 1 mit den Konzentraten 5, 205a, 205b der vorangehenden Ausführungsformen 1, 201 identisch sein und einen geringen Beitrag zur elektrischen Leitfähigkeit der Lösung liefern.

Ein Port 606 verbindet das Innere 610 des Beutels über ein erstes Ende 606b in der Füllkammer 610 des Beutels und einem weiteren Ende 606a außerhalb des Beutels 601 mit dem Äußeren des Beutels. Port 606 ist vorzugsweise durch Schweißverbindungen fluiddicht in der umfänglichen Trennlinie 608, die bevorzugt eine permanente Schweißnaht ist, befestigt. Vorzugsweise befindet sich am Ende 606b des Ports 606 ein Mittel 606c zur Erzeugung von Fluidturbulenzen des zuströmenden Verdünnungsmittel. Dieses Mittel kann als Turbulenz erzeugende Düse ausgestaltet sein oder als eine Turbulenz erzeugende Fritte. Vorzugsweise besteht Port 606 aus einem Schlauch, der den Beutel im Inneren in seiner länglichen Ausdehnung durchmisst. Es wird so im Befüllverfahren gewährleistet, dass zum Beispiel bei aufrechter Lagerung durch Aufnahme des Beutels an einer integralen Halteschiene 611 der Beutel von unten befüllt wird und die Konzentratkammern A, B, C in der Abfolge B, A, C oder A, B, C durch den inneren Fülldruck geöffnet werden.

Die Kammern A, B, C werden durch eine Umfassungslinie 609 umschlossen, die in Abschnitten aus einem Teil der Beutel Umfassungslinie 608 und zum weiteren Teil aus einer Trennlinie gebildet ist, die wenigstens teilweise semipermanente Abschnitte aufweist. Vorzugsweise besteht die erste Trennlinie der Umfassungslinie 609 aus einer Peelnaht und begrenzt in einem Abschnitt 612 zwei Konzentratkammern. Dadurch wird gewährleistet, dass ein peelbarer Abschnitt beim Lösen der Peelnaht zwei Konzentrate freisetzen kann. Die Inhalte der Konzentratkammern A, B, C werden durch weitere Trennlinien 609a, 609b voneinander getrennt. Die weitere Trennlinie 609a ist wenigstens abschnittsweise semipermanent und ist bevorzugt eine vollständig semipermanente Schweißlinie, wie zum Beispiel eine Peelnaht. In der einen dargestellten bevorzugten Ausführungsform bilden die Peelnähte der Trennlinien aus 609, 609a, 609b eine integrale Konstruktion aus Peelnahtabschnitten, die ineinander übergehen, so dass sich ein beginnender Peelnahtbruch der Trennlinie aus 609 auch auf die Trennlinie 609a und 609b fortsetzt.

Das Konzentrat 604 in Konzentratkammer B kann nach Tabelle 1 mit den Konzentraten 4, 204a, 204b der vorangehenden Ausführungsformen 1, 201 identisch sein und keinen Beitrag zur elektrischen Leitfähigkeit der Lösung liefern.

Das Konzentrat 603 in Konzentratkammer C kann nach Tabelle mit den Lösungskomponenten 3, 203a, 203b der vorangehenden Ausführungsformen 1, 201 identisch sein und einen Beitrag zur elektrischen Leitfähigkeit der Lösung liefern.

Durch Befüllen des Beutels 601 durch den Port 606 mit Verdünnungsmittel strömt das Verdünnungsmittel in eine Füllkammer 610. Bei zunehmendem Füllgrad wirkt auf die Trennlinie 609 eine Zugspannung, die dazu führt, dass die Trennlinie 609 teilweise gelöst und das Konzentrat 604 und oder gleichzeitig Konzentrat 605 mit dem Verdünnungsmittel vermischt wird. Bei weiterer Befüllung öffnet sich auch die Konzentratkammer A, sofern sie nach der gezeigten Ausführung nicht gleichzeitig mit Kammer B geöffnet wird, durch Lösen der Trennlinie 609a. Vorzugsweise besteht Konzentrat 605 aus Substanzen, die einen geringen Beitrag zur elektrischen Leitfähigkeit der Lösung beitragen. Durch weiteres Befüllen des Beutels 601 löst sich die Trennlinie aus 609 und nachfolgend die Trennlinie 609a, 609b vollständig, so dass Konzentrat 603 mit dem Verdünnungsmittel und Konzentraten 605 und 604 vermischt wird. Die Mengen an Konzentraten und Verdünnungsmitteln sind so bemessen, dass eine fertige physiologisch annehmbare Lösung, insbesondere Dialyselösung entsteht.

Ein weiterer Port 607 mit einem ersten Ende 607a außerhalb des Beutels und einem weiteren Ende 607b innerhalb des Beutels dient zur Entnahme oder zur Zuführung von Lösung, Substanzen, Medikamenten etc.

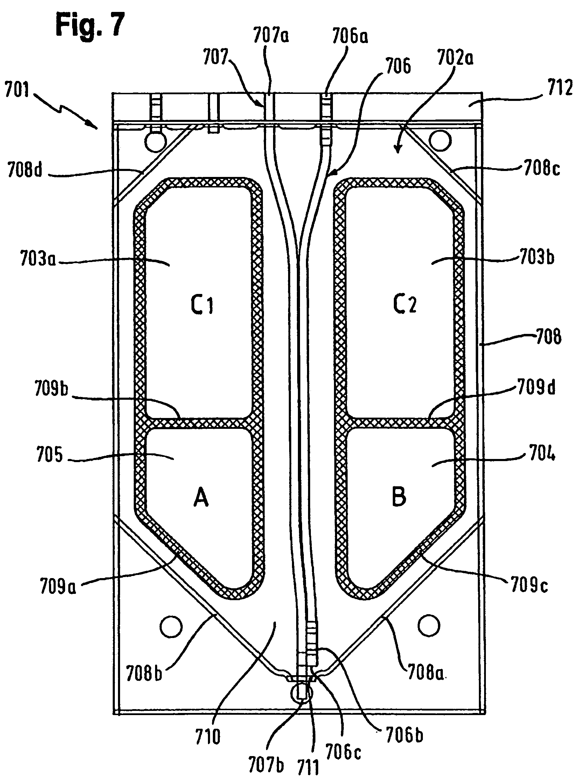

**Figur 7** zeigt eine weitere Ausführungsform, die ähnlich der Ausführungsform nach Fig. 2 ist. Der Container weist in Perspektive zur Zeichenebene eine obere Außenwandung 702a und eine untere nicht gezeigte Außenwandung 702b auf, die durch einen gemeinsamen umfänglichen Rand 708 begrenzt und fluiddicht verbunden sind. In einer bevorzugten Version sind 702a und 702b die oberen und unteren Folienblätter eines Beutels, 708 wird durch eine permanente Schweißnaht gebildet.

Der Beutel verfügt über eine erste Konzentratkammer A mit Konzentraten 705, die in einer beispielhaften Ausführung aus Substanzen bestehen, die einen Beitrag zur elektrischen Leitfähigkeit in Lösung liefern. Nach den beispielhaften Ausführungen der Tabelle 1 befinden sich in der Konzentratkammer A das Konzentrat 705, die Substanzen Magnesiumcarbonat oder allgemein lösliche Magnesiumsalze, Calciumsalze, zum Beispiel gelöst oder in fester Form, Magnesiumchlorid und/oder Calciumchlorid, Zitronesäure oder Zitratsalze, Säuresalze oder Säuren in fester oder in gelöster Form.

Eine zweite Konzentratkammer C1 mit Konzentrat 703a enthält in einer beispielhaften Ausführung Substanzen, die einen Beitrag zur elektrischen Leitfähigkeit in Lösung beitragen. In einer beispielhaften Ausführung kann es sich dabei um Natriumchlorid und/oder Bikarbonat oder allgemein um lösliche Salze der Kohlensäure, zum Beispiel Natriumcarbonat oder anderen physiologisch verträglichen Puffern, zum Beispiel Salze von schwachen Säuren handeln.

Die Konzentratkammern A und C1 werden entlang einer geschlossene Umfassungslinie von einer Trennlinie 709a umschlossen, die in der dargestellten Ausführungsform aus einer wenigstens abschnittsweise semipermanenten Trennlinie besteht. In einer Ausführungsform besteht die erste Trennlinie aus 709a aus einer Peelnaht. Die Inhalte der Konzentratkammern A und C1 werden durch die weitere Trennlinie 709b, voneinander getrennt. Die weitere Trennlinie 709b kann aus einer permanenten Schweißlinie, einer teilweise semipermanenten Schweißlinie oder einer Peelnaht bestehen. Vorzugsweise sind die Trennlinien 709a und 709b als Peelnähte ausgestaltet und bilden eine zusammenhängende integrale Konstruktion von Peelnahtabschnitten.

Die beispielhafte Ausführung nach Fig. 7 ist weiterhin dadurch gekennzeichnet, dass ein Verbindungsport 706 die Füllkammer 710 des Beutels über ein erstes Ende im Inneren des Beutels 706b und einem weiteren Ende 706a außerhalb des Beutels 701 mit dem Äußeren des Beutels verbindet. Port 706 ist vorzugsweise durch Schweißverbindungen fluiddicht in der umfänglichen Trennlinie 708 befestigt. Vorzugsweise befindet sich am Ende 706b des Ports 706 ein Mittel 706c zur Erzeugung von Fluidturbulenzen des zuströmenden Verdünnungsmittels. Dieses Mittel kann als Turbulenz erzeugende Düse ausgestaltet sein oder als eine Turbulenz erzeugende Fritte. Weiterhin besteht Port 706 aus einem Schlauch, der den Beutel im Inneren in seiner länglichen Ausdehnung durchmisst. Es wird so im Befüllverfahren gewährleistet, dass bei aufrechter Lagerung der Beutel, zum Beispiel durch Aufnahme des Beutels an einer oberen Halteschiene 712, der Beutel von unten befüllt wird und die Konzentratkammern A, B, C1, C2 in der Abfolge A gleichzeitig mit B, vor C1 gleichzeitig mit C2 durch den inneren Fülldruck geöffnet werden.

Ein weiterer Port 707 mit einem ersten Ende 707a außerhalb des Beutels und einem weiteren Ende 707b dient zur Rückführung verbrauchter medizinischer Flüssigkeit, bevorzugt Dialyselösung. Port 707 ist dabei im Inneren des Beutels als Schlauch ausgebildet und dafür vorgesehen, bei hängender Lagerung des Beutels, zum Beispiel durch Aufnahme des Beutels an der oberen Halteschiene 712, den Beutel entlang einer länglichen Ausdehnung zu durchmessen. An der Stelle 711 durchdringt der Schlauch 707 die Beutel Umfassungslinie 708 des Beutels 701 und mündet in eine weitere, nicht gezeigte Kammer. 711 kann dabei eine fluiddichtende Schweißstelle sein, die den Beutel zwischen den oberen und unteren Begrenzungsebenen 702a und 702b fixiert und Teil der geschweißten Umfassungslinie 708 ist. Die nicht dargestellte Kammer kann ein umhüllender Container, bevorzugt Beutel, sein, der integraler Bestandteil des Containers 701 sein kann. Es ergibt sich so eine "bag in bag" Konstruktion bei der der Beutel, der die gebrauchsfertige Flüssigkeit aufnimmt, von einem Beutel, der die verbrauchte Flüssigkeit aufnimmt, umhüllt wird. Ebenso kann die nicht gezeigte Kammer aber auch separat ausgebildet sein.

Weiterhin zeigt die Ausführung in Fig. 7 einen zweiten Satz von Konzentratkammern B, C2, die durch eine weitere Trennlinie entlang einer geschlossenen Umfassungslinie 709c umschlossen sind. Eine weitere Trennlinie 709d trennt die Inhalte der Konzentratkammern B und C2. In einer bevorzugten Ausführungsform bilden die Peelnähte 709c und 709d eine integrale Konstruktion aus Peelnahtabschnitten, die ineinander übergehen.

In der beispielhaften Ausführungsform besteht das Konzentrat 704 in Kammer B, aus Substanzen, die keinen Beitrag zur elektrischen Leitfähigkeit in Lösung aufweisen. In einer beispielhaften Ausführung nach Tabelle 1 kann es sich dabei um wasserfreie Glukose handeln.

Das Konzentrat 705 in Konzentratkammer A kann mit den Konzentratkammern 205a der Ausführung aus Fig. 2 identisch sein oder nur einen Teil der Komponenten 205a enthalten. In einer beispielhaften Ausführung besteht das Konzentrat 705 aus Magnesiumcarbonat oder allgemein aus löslichen Magnesiumsalzen, Calciumchlorid oder allgemein aus löslichen Kalziumsalzen, Zitronesäure oder Zitratsalzen oder einer anderen Säure.

Eine weitere Konzentratkammer C2 mit Konzentrat 703b enthält weitere Substanzen, die mit den Substanzen der Konzentrate 705, 704 unverträglich sind, also zur Degradation neigen oder unerwünschte Wechselwirkung eingehen. Darüber hinaus weist Konzentrat 703b in Lösung einen Beitrag zur elektrischen Leitfähigkeit auf. Es wird so sichergestellt, dass mit Zunahme der elektrischen Leitfähigkeit durch Lösung der Komponenten 703b auch die Lösung der Komponenten 704 erfolgt ist, da die Lösungskomponenten in den Kammern B und C2 über ein zusammenhängendes zu öffnendes Peelnahtsystem begrenzt und getrennt sind.

Die Ausführungsform zeigt weiterhin eine Umfassungslinie 708, die vorzugsweise aus einer permanenten Schweißnaht besteht. Zusätzliche permanente Schweißlinien, Abschnitte 708a und 708b begrenzen den Containerinhalt oder Beutelinhalt so, dass ein schräger Boden des Innenraums entsteht. Die Konstruktion begünstigt die Verwirbelung des zuströmenden Verdünnungsmittels durch Fluidturbulenz erzeugendes Mittel 706c und damit den Lösungsprozess der Komponenten 705, 704, 703a, 703b. Begrenzungslinien 708c und 708d verleihen dem gefüllten Container, insbesondere Beutel, weitere Stabilität im gefüllten Zustand. Dies ist insbesondere für großvolumige Container von Bedeutung, bei denen der Innendruck durch die beinhalteten Mengen belastende Spannungswirkung auf die Umfassungslinie 708 ausüben kann. Großvolumige Beutel sind in diesem Sinne als Container zu verstehen, die ein Volumen von 5 bis 120 Liter, 40 bis 80 Litern, insbesondere 60 Liter ± 15% aufweisen.

**Figur 8a** zeigt schematisch in einem seitlichen Querschnitt ein erfindungsgemäßes Containersystem mit einem inneren Beutel (830a), der Konzentratkammern (831 a) aufweist und einem äußeren Beutel (833a), wobei die erste Folie des inneren Beutels tief gezogene Bereiche (832a) aufweist.

**Figur 8b** zeigt schematisch in einem seitlichen Querschnitt ein erfindungsgemäßes Containersystem mit einem inneren Beutel (830b) und einem äußeren Beutel (833b), wobei die erste Folie (836b) des inneren Beutels tief gezogene Bereiche (832b) aufweist und die zweite Folie (837b) nicht tief gezogen ist und wobei die Konzentratkammern des inneren Beutels bereits geöffnet sind. Figur 8b zeigt einen Zustand, in dem die Konzentratkammern idealisiert in einem geöffneten Zustand vorliegen. D.h. die Trennlinien zwischen den Konzentratkammern wurden durch das Befüllen von der Bodenseite (835b) mit einem Verdünnungsmittel bereits geöffnet. Im weiteren Befüllungsvorgang werden die erste Folie (836b) und die zweite Folie (837b) gedehnt. Die zweite Folie dehnt sich dabei im gleichen Maße wie die tief gezogene erste Folie, wenn die Folie entsprechend dünner ausgebildet ist.

**Figur 8c** zeigt ein erfindungsgemäßes Containersystem aus einem inneren Beutel (831 c) aus einer ersten Folie (836c) mit tief gezogenen Bereichen (832b) und einer zweiten Folie (837c) mit tief gezogenen Bereichen (834c), und einen umhüllenden Beutel (833c). Die tief gezogenen Bereiche der beiden Folien sind gegenüberliegend. Erfolgt eine Befüllung des Beutels von der unteren Bodenseite (835c), die zur Dehnung des Folienmaterials führt, so werden beide Folien gleichmäßig gedehnt. Der Beutel erhält somit bei der Befüllung keine einseitigen Auswölbungen, die die Funktion des Beutels beeinträchtigen könnten.

## Patentansprüche

1. Container (1, 201, 401,501, 601, 701) geeignet für die Herstellung medizinischer Lösungen, bestehend aus permanenten Außenwandungen (2a, 2b, 202a, 202b, 402a, 402b, 502a, 502b, 702a, 702b), die ein inneres Fassungsvolumen des Containers definieren, enthaltend
• wenigstens eine Füllkammer (10, 210, 510, 710), wenigstens eine erste Konzentratkammer (4, 204a, 204b, 504, 704, 705) und eine zweite Konzentratkammer (3, 203 a, 203b, 503, 703a, 703b), die im Inneren des Containers angeordnet sind,
• wobei die Konzentratkammern (3, 4, 5, 203 a, 203b, 204a, 204b, 503, 504, 703a, 703b, 704, 705) durch wenigstens abschnittsweise semipermanente Trennlinien (9, 9a, 9b, 209a, 209b, 209d, 209e, 209f, 509, 509b, 709a, 709b, 709c, 709d), die mit den Außenwandungen in Verbindung stehen, begrenzt sind,
• darunter wenigstens eine erste Trennlinie (9, 209a, 209d, 409, 509, 609, 709a, 709c), die die Füllkammer von der einen ersten und der einen zweiten Konzentratkammer trennt, die nicht mit der Umfassungslinie (8, 208, 208a, 208b, 508, 708) des Containers zusammenwirkt und zumindest die eine erste Konzentratkammer (4, 204a, 204b, 704, 705) und die eine zweite Konzentratkammer (3, 203 a, 203b, 703a, 703b), entlang einer geschlossenen Linie umschließt.

2. Container (1, 201, 401,501, 601, 701) nach Anspruch 1, **dadurch gekennzeichnet, dass** die eine Füllkammer (10, 210, 410, 510, 610, 710) mit einem Zuführport (6, 206, 406, 506, 606, 706) des Containers (1, 201, 401,501, 601, 701) verbunden ist.

3. Container (1, 201, 401,501, 601, 701) nach Anspruch 1 oder 2, **dadurch gekennzeichnet, dass** der Container eine dritte Konzentratkammer optional eine vierte Konzentratkammer, optional eine fünfte Konzentratkammer, optional eine sechste Konzentratkammer, optional eine siebte Konzentratkammer, optional eine achte Konzentratkammer umfasst.

4. Container (1, 201,501) nach Anspruch 3, **dadurch gekennzeichnet, dass** die eine erste Trennlinie, eine erste Konzentratkammer (4, 204a, 204b, 504), eine zweite Konzentratkammer (3, 203 a, 203b, 503) und eine dritte Konzentratkammer umschließt (5, 205a, 205b, 505).

5. Container (1, 201, 501, 701) nach einem oder mehreren der Ansprüche 1 bis 4, **dadurch gekennzeichnet, dass** der Container ein Beutel ist.

6. Container (1, 201, 501, 701) nach einem oder mehreren der Ansprüche 1 bis 5, **dadurch gekennzeichnet, dass** die eine erste Trennlinie (9, 209a, 209d, 409, 509, 609, 709a, 709c), eine Schweißnaht ist, die zumindest abschnittsweise als eine Peelnaht ausgestaltet ist oder durchgehend als Peelnaht ausgestaltet ist.

7. Container (1, 201, 401, 501, 601, 701) nach Anspruch 6, wobei die Peelnaht eine Festigkeit von 0,1 bis 8 N/15 mm hat.

8. Container nach Anspruch 6 oder 7, **dadurch gekennzeichnet, dass** die eine erste Konzentratkammer (4, 204a, 204b, 504, 704, 705) und die eine zweite Konzentratkammer (3, 203a, 203b, 503, 703a, 703b von einer zweiten Trennlinie (9b, 209c, 209f, 509b, 709b, 709d) gegeneinander getrennt sind.

9. Container (1, 201, 401, 501, 601, 701) nach Anspruch 8, wobei die eine erste Trennlinie (9, 209a, 209d, 409, 509, 609, 709a, 709c) und die eine zweite Trennlinie (9b, 209c, 209f, 509b, 709b, 709d) als integrale Peelnahtkonstruktion ausgebildet sind.

10. Container (1, 201, 501,) nach Anspruch 4 **dadurch gekennzeichnet, dass** die eine zweite Konzentratkammer und die eine dritte Konzentratkammer von einer dritten Trennlinie (9a, 209b, 209e, 509a,) gegeneinander getrennt sind.

11. Container (1, 201,) nach Anspruch 4 **dadurch gekennzeichnet, dass** die eine erste Trennlinie (9, 209a, 209d, 609), die eine zweite Trennlinie (9b, 209c, 209f,) und die eine dritte Trennlinie (9a, 209b, 209e,) als integrale Peelnahtkonstruktion ausgebildet sind.

12. Container (1, 201, 501, 701) nach einem der vorhergehenden Ansprüche, **dadurch gekennzeichnet, dass** der Container aus elastisch dehnbarem Folienmaterial hergestellt ist.

13. Container (1, 201, 501, 701) nach einem der vorhergehenden Ansprüchen, **dadurch gekennzeichnet, dass** die eine erste Konzentratkammer (4, 204a, 204b, 504, 704, 705) ein Konzentrat (B) enthält, das keinen wesentlichen Beitrag zur elektrischen Leitfähigkeit in Lösung liefert.

14. Container (1, 201, 501, 701) nach Anspruch13, **dadurch gekennzeichnet, dass** das Konzentrat (B) der einen ersten Konzentratkammer (4, 204a, 204b, 504, 704, 705), die keinen wesentlichen Beitrag zur elektrischen Leitfähigkeit liefert, Glukose umfasst.

15. Container (1, 201, 501, 701) nach einem oder mehreren der einem der vorhergehenden Ansprüche, **dadurch gekennzeichnet, dass** die eine zweite Konzentratkammer (3, 203 a, 203b, 403, 503, 603, 703a, 703b) ein Konzentrat (C, C1, C2) umfasst, das einen Beitrag zur elektrischen Leitfähigkeit in Lösung liefert.

16. Verfahren zum Herstellen einer medizinischen Lösung aus mehreren Konzentraten (A, B, B₁, B₂, C, C₁, C₂), unter Verwendung eines Containers nach einem oder mehreren der Ansprüche 1 bis 15, umfassend die Schritte:
• Einleiten von Flüssigkeit in eine Füllkammer (10, 210, 410, 510, 610, 710) des Containers (1, 201,401,501,601,701)
• Aufbrechen einer semipermanenten Trennlinie (9, 209a, 209d, 409, 509, 609, 709a, 709c), die eine erste Konzentratkammer (4, 204a, 204b, 404, 504, 604, 704, 705) begrenzt durch den sich bildenden hydrostatischen Druck
• Freisetzen von Konzentraten (A, B, B₁, B₂), die im Wesentlichen keinen Beitrag zur elektrischen Leitfähigkeit der Lösung liefern
• Weiteres Füllen und Ausüben von Druck und gleichzeitiges oder zeitlich versetztes weiteres Aufbrechen der Trennlinie (9, 209a, 209d, 509, 609, 709a, 709c) oder einer weiteren Trennlinie (409b), die eine weitere Konzentratkammer (3, 203 a, 203b, 403, 503, 603, 703a, 703b), begrenzt
• Freisetzen von Konzentrat (C, C₁, C₂) der einen weiteren Konzentratkammer, die einen Beitrag zur elektrischen Leitfähigkeit der Lösung liefert.

## Claims

1. A container (1, 201, 401, 501, 601, 701) suitable for preparing medicinal solutions consisting of permanent outside walls (2a, 2b, 202a, 202b, 402a, 402b, 502a, 502b, 602a, 602b, 702a, 702b) which define an internal capacity of the container, comprising
- at least one filling chamber (10, 210, 510, 710), at least one first concentrate chamber (4, 204a, 204b, 504, 704, 705) and a second concentrate chamber (3, 203a, 203b, 503, 703a, 703b) which are arranged in an interior of the container,
- the concentrate chambers (3, 4, 5, 203a, 203b, 204a, 204b, 503, 504, 703a, 703b, 704, 705) being separated by dividing lines (9, 9a, 9b, 209a, 209b, 209d, 209e, 209f, 509, 509b, 709a, 709b, 709c, 709d) which are semipermanent in at least some sections and are connected to the outside walls,
- including at least one first dividing line (9, 209a, 209d, 409, 509, 609, 709a, 709c), which separates the filling chamber from the one first and the one second concentrate chamber which does not interact with the circumferential line (8, 208, 208a, 208b, 508, 708) of the container and which surrounds at least the one first concentrate chamber (4, 204a, 204b, 704, 705) and the one second concentrate chamber (3, 203a, 203b, 703a, 703b) along a closed line.

2. The container (1, 201, 401, 501, 601, 701) according to Claim 1, **characterized in that** the one filling chamber (10, 210, 410, 510, 610, 710) is connected with a feed port (6, 206, 406, 506, 606, 706) of the container (1, 201, 401, 501, 601, 701).

3. The container (1, 201, 401, 501, 601, 701) according to Claim 1, **characterized in that** the container comprises a third concentrate chamber, optionally a fourth concentrate chamber, optionally a fifth concentrate chamber, optionally a sixth concentrate chamber, optionally a seventh concentrate chamber, optionally an eighth concentrate chamber.

4. The container (1, 201, 501) according to Claim 3, **characterized in that** the one first dividing line surrounds a first concentrate chamber (4, 204a, 204b, 504), a second concentrate chamber (3, 203a, 203b, 503) and a third concentrate chamber (5, 205a, 205b, 505).

5. The container (1, 201, 501, 701) according to one or more of Claims 1 to 4, **characterized in that** the container is a bag.

6. The container (1, 201, 501, 701) according to one or more of Claims 1 to 5, **characterized in that** the one first dividing line (9, 209a, 209d, 409, 509, 609, 709a, 709c) is a weld configured as a peel seam in at least some sections or uniformly configured as a peel seam.

7. The container (1, 201, 401, 501, 601, 701) according to Claim 6, wherein the peel seam has a strength of from 0.1 to 8 N/15 mm.

8. The container according to Claim 6 or 7, **characterized in that** the one first concentrate chamber (4, 204a, 204b, 504, 704, 705) and the one second concentrate chamber (3, 203a, 203b, 503, 603, 703a, 703b) are separated from one another by a second dividing line (9b, 209c, 209f, 509b, 709b, 709d).

9. The container (1, 201, 401, 501, 601, 701) according to Claim 8, wherein the one first dividing line (9, 209a, 209d, 409, 509, 609, 709a, 709c) and the one second dividing line (9b, 209c, 209f, 509b, 709b, 709d) are configured as integral peel seam.

10. The container (1, 201, 501) according to Claim 4, **characterized in that** the one second concentrate chamber and the one third concentrate chamber are separated from one another by a third dividing line (9a, 209b, 209e, 509a).

11. The container (1, 201) according to Claim 4, **characterized in that** the one first dividing line (9, 209a, 209d, 609), the one second dividing line (9b, 209c, 209f) and the one third dividing line (9a, 209b, 209e) are designed as an integral peel seam construction.

12. The container (1, 201, 501, 701) according to any one of the preceding claims, **characterized in that** the container has a material of construction that is an elastically extensible film material.

13. The container (1, 201, 501, 701) according to any one of the preceding claims, **characterized in that** the one first concentrate chamber (4, 204a, 204b, 504, 704, 705) contains a concentrate (B) which does not make any contribution toward an electrical conductivity in solution.

14. The container (1, 201, 501, 701) according to Claim 13, **characterized in that** the concentrate (B) of the one first concentrate chamber (4, 204a, 204b, 504, 704, 705) which does not make any contribution toward an electrical conductivity comprises glucose.

15. The container (1, 201, 401, 501, 601, 701) according to any one or more of the preceding claims, **characterized in that** the one second concentrate chamber (3, 203a, 203b, 403, 503, 603, 703a, 703b) contains a concentrate (C, C1, C2) which makes a contribution toward the electrical conductivity in solution.

16. A method for preparing a medicinal solution from multiple concentrates (A, B, B₁, B₂, C, C₁, C₂) using a container to any one or more of Claims 1 to 15, comprising the steps:
- introducing liquid into a filling chamber (10, 210, 410, 510, 610, 710) of the container (1, 201, 401, 501, 601, 701),
- breaking up a semipermanent dividing line (9, 209a, 209d, 409, 509, 609, 709a, 709c) which borders a first concentrate chamber (4, 204a, 204b, 404, 504, 604, 704, 705) due to developing hydrostatic pressure,
- releasing the concentrates (A, B, B₁, B₂) which make essentially no contribution toward an electrical conductivity of the medicinal solution,
- additional filling and application of pressure and breaking open of the dividing line (9, 209a, 209d, 509, 609, 709a, 709c) or another dividing line (409b) which borders another concentrate chamber (3, 203a, 203b, 403, 503, 603, 703a, 703b) at a same time or with a time offset, and
- releasing the concentrate (C, C₁, C₂) of the one additional concentrate chamber which makes a contribution toward the electrical conductivity of the solution.

## Revendications

1. Récipient (1, 201, 401, 501, 601, 701) convenant à la fabrication de solutions médicales, constitué de parois externes permanentes (2a, 2b, 202a, 202b, 402a, 402b, 502a, 502b, 702a, 702b) qui définissent un volume de contenance interne du récipient, contenant
• au moins une chambre de remplissage (10, 210, 510, 710), au moins une première chambre de concentré (4, 204a, 204b, 504, 704, 705) et une deuxième chambre de concentré (3, 203 a, 203b, 503, 703a, 703b) qui sont disposées à l'intérieur du récipient,
• dans lequel les chambres de concentré (3, 4, 5, 203 a, 203b, 204a, 204b, 503, 504, 703a, 703b, 704, 705) sont limitées par des lignes de séparation (9, 9a, 9b, 209a, 209b, 209d, 209e, 209f, 509, 509b, 709a, 709b, 709c, 709d) semi-permanentes au moins par section qui sont connectées aux parois externes,
• au moins une première ligne de séparation (9, 209a, 209d, 409, 509, 609, 709a, 709c) parmi elles qui sépare la chambre de remplissage d'une des premières et d'une des deuxièmes chambres de concentré, qui ne coopère pas avec la ligne de pourtour (8, 208, 208a, 208b, 508, 708) du récipient et entoure au moins une des premières chambres de concentré (4, 204a, 204b, 704, 705) et une des deuxièmes chambres de concentré (3, 203 a, 203b, 703a, 703b) le long d'une ligne fermée.

2. Récipient (1, 201, 401, 501, 601, 701) selon la revendication 1, **caractérisé en ce qu'**une des chambres de remplissage (10, 210, 410, 510, 610, 710) est reliée à un port d'amenée (6, 206, 406, 506, 606, 706) du récipient (1, 201, 401, 501, 601, 701).

3. Récipient (1, 201, 401, 501, 601, 701) selon la revendication 1 ou 2, **caractérisé en ce que** le récipient comprend une troisième chambre de concentré, en option une quatrième chambre de concentré, en option une cinquième chambre de concentré, en option une sixième chambre de concentré, en option une septième chambre de concentré, en option une huitième chambre de concentré.

4. Récipient (1, 201, 501) selon la revendication 3, **caractérisé en ce qu'**une des premières lignes de séparation entoure une première chambre de concentré (4, 204a, 204b, 504), une deuxième chambre de concentré (3, 203 a, 203b, 503) et une troisième chambre de concentré (5, 205a, 205b, 505).

5. Récipient (1, 201, 501, 701) selon une ou plusieurs des revendications 1 à 4, **caractérisé en ce que** le récipient est un sachet.

6. Récipient (1, 201, 501, 701) selon une ou plusieurs des revendications 1 à 5, **caractérisé en ce qu'**une des premières lignes de séparation (9, 209a, 209d, 409, 509, 609, 709a, 709c) est une soudure qui est configurée au moins par section en tant que joint pelable ou est configurée en continu en tant que joint pelable.

7. Récipient (1, 201, 401, 501, 601, 701) selon la revendication 6, dans lequel le joint pelable a une résistance de 0,1 à 8 N/15 mm.

8. Récipient selon la revendication 6 ou 7, **caractérisé en ce qu'**une des premières chambres de concentré (4, 204a, 204b, 504, 704, 705) et une des deuxièmes chambres de concentré (3, 203a, 203b, 503, 703a, 703b) sont séparées l'une contre l'autre par une deuxième ligne de séparation (9b, 209c, 209f, 509b, 709b, 709d).

9. Récipient (1, 201, 401, 501, 601, 701) selon la revendication 8, dans lequel une des premières lignes de séparation (9, 209a, 209d, 409, 509, 609, 709a, 709c) et une des deuxièmes lignes de séparation (9b, 209c, 209f, 509b, 709b, 709d) sont réalisées en tant que construction de joint pelable intégrale.

10. Récipient (1, 201, 501) selon la revendication 4, **caractérisé en ce qu'**une des deuxièmes chambres de concentré et une des troisièmes chambres de concentré sont séparées l'une contre l'autre par une troisième ligne de séparation (9a, 209b, 209e, 509a).

11. Récipient (1, 201) selon la revendication 4, **caractérisé en ce qu'**une des premières lignes de séparation (9, 209a, 209d, 609), une des deuxièmes lignes de séparation (9b, 209c, 209f) et une des troisièmes lignes de séparation (9a, 209b, 209e) sont réalisées en tant que construction de joint pelable intégrale.

12. Récipient (1, 201, 501, 701) selon une des revendications précédentes, **caractérisé en ce que** le récipient est fabriqué en matériau de film extensible élastiquement.

13. Récipient (1, 201, 501, 701) selon une des revendications précédentes, **caractérisé en ce qu'**une des premières chambres de concentré (4, 204a, 204b, 504, 704, 705) contient un concentré (B) qui ne contribue essentiellement pas à la conductivité électrique en solution.

14. Récipient (1, 201, 501, 701) selon la revendication 13, **caractérisé en ce que** le concentré (B) d'une des premières chambres de concentré (4, 204a, 204b, 504, 704, 705) qui ne contribue essentiellement pas à la conductivité électrique comprend du glucose.

15. Récipient (1, 201, 501, 701) selon une ou plusieurs des revendications précédentes, **caractérisé en ce qu'**une des deuxièmes chambres de concentré (3, 203 a, 203b, 403, 503, 603, 703a, 703b) comprend un concentré (C, C1, C2) qui contribue à la conductivité électrique en solution.

16. Procédé de fabrication d'une solution médicale à partir de plusieurs concentrés (A, B, B₁, B₂, C, C₁, C₂) en utilisant un récipient selon une ou plusieurs des revendications 1 à 15, comprenant les étapes :
• introduction de liquide dans une chambre de remplissage (10, 210, 410, 510, 610, 710) du récipient (1, 201, 401, 501, 601, 701)
• rupture d'une ligne de séparation semi-permanente (9, 209a, 209d, 409, 509, 609, 709a, 709c) qui limite une première chambre de concentré (4, 204a, 204b, 404, 504, 604, 704, 705) par la pression hydrostatique qui se forme
• libération de concentrés (A, B, B₁, B₂) qui ne contribuent essentiellement pas à la conductivité électrique de la solution
• remplissage supplémentaire et exercice de pression et rupture supplémentaire simultanée ou décalée dans le temps de la ligne de séparation (9, 209a, 209d, 509, 609, 709a, 709c) ou d'une ligne de séparation supplémentaire (409b) qui limite une chambre de concentré supplémentaire (3, 203 a, 203b, 403, 503, 603, 703a, 703b)
• libération de concentré (C, C₁, C₂) d'une des chambres de concentré supplémentaires qui contribue à la conductivité électrique de la solution.
